# EUROPEAN PATENT SPECIFICATION

(11) **EP 1 068 530 B1**
(45) Date of publication and mention of the grant of the patent: **25.05.2005**
(21) Application number: 99911137.0
(22) Date of filing: 02.03.1999
(51) Int. Cl.: G01N 33/53, C07K 16/18, C07K 16/28, C07K 16/30, C07H 21/04, C12N 1/02, C12N 15/63, G01N 33/574

(54) **DIAGNOSTIC FOR METASTATIC PROSTATE CANCER**
DIAGNOSE FÜR METASTASISCHEN PROSTATAKREBS
DIAGNOSTIC DU CANCER DE LA PROSTATE METASTATIQUE

(30) Priority: 03.03.1998 US 76664 P
(43) Date of publication of application: 17.01.2001
(73) Proprietor: Emory University, Atlanta, GA 30322 (US)
(72) Inventor: WU, Guang-Jer, Atlanta, GA 30345 (US)
(74) Representative: Fisher, Adrian John
(86) International application number: PCT/US1999/004850
(87) International publication number: WO 1999/045392

(56) References cited:
- WO-A-96/34117
- WO-A-97/28193
- LEHMANN J.G. et al., "MUC18, a Marker of Tumor Progression in Human Melanoma, Shows Sequence Similarity to the Neural Cell Adhesion Molecules of the Immunoglobulin Superfamily", PROC. NATL. ACAD. SCI. USA, December 1989, Vol. 86, pages 9891-9895, XP002920850
- LUCA M. et al., "Direct Correlation Between MUC18 Expression and Metastatic Potential of Human Melanoma Cells", MELANOMA RESEARCH 1993, Vol. 3, pages 35-41, XP002920851

## Description

### BACKGROUND OF THE INVENTION

Prostate cancer accounts for about 1 in 10 cancer cases in the United States, and it is the most often diagnosed cancer in males [Henderson et al. (1991) *Science* 254, 1131-1138]. While in many affected patients, the tumors are slow-growing and nonmetastatic, in others the malignant prostate tumors are aggressive and metastasize. When prostate cancer metastasizes, the prognosis for the patient is poor, especially without treatment.

To date, the most frequently used test for prostate cancer is the serum level of prostate specific antigen (PSA) and the radionuclide bone scan for detecting prostate cancer metastases before definitive therapy is initiated. However, the elevated level of PSA in serum is not predictive of the pathologic stage of the prostate cancer or the presence of metastatic disease. PSA, a serine protease, is not exclusively expressed in the epithelial cells of metastatic prostate cancer, but it is also expressed in normal epithelial cells, primary tumors and benign prostate hyperplasia.

The altered expression of cell-adhesion molecules has been correlated with metastasis of many cancers. Low or no expression of E-cadherin, a cell-adhesion molecule, has been found in high-grade prostate carcinoma, and this indicates a poor prognosis for those prostate cancer patients. However, the absence of an antigen is not very useful as a diagnostic marker for cancer metastasis.

MUC 18 is a glycoprotein of about 113 kDa which serves as a cell adhesion molecule on the surface of melanoma cells, and it has been correlated with the ability of melanomas to metastasize [See, e.g., Lehmann et al. (1989) *Proc. Nat. Acad. Sci. USA* 86, 9891-9895; Luca et al. (1993) *Melanoma Res.* 3, 35-41; Johnson et al. (1996) *Curr. Top. Microbiol. Immunol*. 213, 95-105; Xie et al. (1997) *Cancer Res.* 57, 2295-2303; Tang and Honn (1994-1995) *Invasion Metas*. 14, 109-122; Rummel et al. (1996) *Cancer Res.* 56, 2218-2223]. MUC18 is also known as MCAM and CD146. MUC18 carries a carbohydrate modification known as HNK-1 or CD57 [Shih et al. (1994) *Cancer Res.* 54, 2514-2520]. Besides being associated with melanoma cells' ability to metastasize, MUC18 is also associated with normal vascular tissue, and on the smooth muscle of venules, and it expresses sporadically on capillary epithelium [Johnson, J. (1994-1995) *Invasion Metas*. 14, 123-130].

There is a longfelt need in the art for an improved diagnostic test for metastatic prostate cancer so that appropriate therapy can be initiated as soon as possible and so that the number of false positive results can be minimized.

### SUMMARY OF THE INVENTION

The present invention provides an improved diagnostic test for prostate cancer which has a relatively high potential for metastasis or which has metastasized. This allows the physician to choose appropriate surgical, chemotherapeutic or radiation treatment regimens. This improved assay is based on the correlation of high levels of expression of the MUC 18 coding sequence as measured by MUC18 mRNA or MUC18 protein. This expression can be detected at the transcriptional level, where mRNA levels are monitored, or detection of the MUC 18 gene product at the translation level can be determined, for example, through the use of an immunoassay for the MUC18 protein. The source of the material for these tests is prostate biopsy tumor tissue (e.g., from a needle biopsy) from a patient needing a determination of the metastatic potential of a prostate tumor or from cells from a prostate tumor.

Relative levels of transcriptional expression (mRNA) of the MUC18 coding sequence can be determined by Northern hybridization analysis or by quantitative reverse transcription polymerase chain reaction (RT-PCR) in normal and neoplastic prostate tissue samples and in biopsy material.

Translational expression of MUC18 can be determined by any of a number of adaptations of an immunoassay using antibody specific for the MUC18 cell surface antigen. The relative level of MUC18 can be determined by standard immunoassays using a MUC18-specific antibody preparation and a detection system suitable for the assay. Immunoassays can include, but are not limited to, immunofluorescence assays, radioimmunoassays, enzyme-linked immunosorbent assays, and Western (immuno) blot assays. In the context of the present invention, relative amounts of the MUC18 protein are determined in tissue samples (e.g., biopsy material).

It is a further object of the present invention to provide an antibody which inhibits prostate cancer metastasis. In particular, antibody specific to MUC18 prevents metastasis of prostate cancer cells.

Additional objects include vectors directing the expression of an immunogenic fragment of human MUC18 and the corresponding recombinantly expressed protein. As specifically exemplified, an immunogenic fragment of human MUC18 is encoded by the PvuII to XhoI fragment within the sequence given in Tables 1A-1B.

### BRIEF DESCRIPTION OF THE DRAWINGS

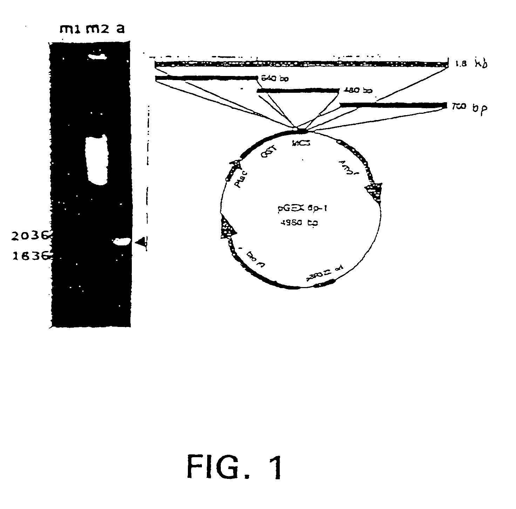

Fig. 1 shows the results for RT-PCR amplified human MUC18 cDNA (left panel) and cloning of the whole cDNA and its fragments into a GST-fusion protein expression vector (right panel). RT-PCR amplification of human MUC18 cDNA from the poly(A)+RNA isolated and purified from a human melanoma cell line, Sk-Mel-28. Left panel: Lane (a) shows the expected PCR product of 1957 bp (as indicated by an arrow head). Lane (m1) shows the I kb ladders and lane (m2) the 123-bp ladders as DNA molecular size markers. Right panel: The plasmid map of the cloned whole human MUC18 cDNA and three fragments in a GST-fusion expression system.

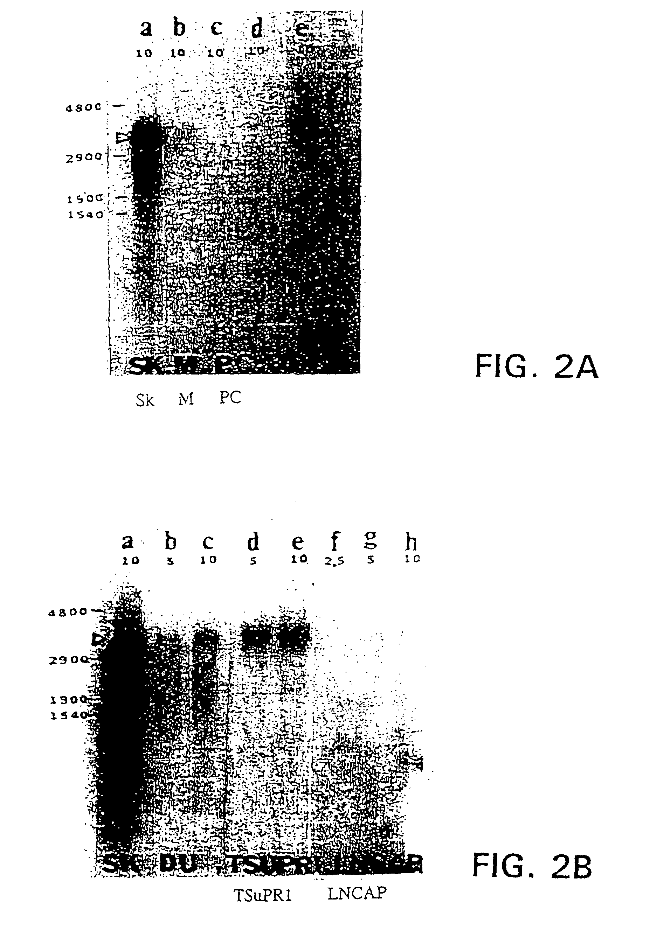

Fig. 2 illustrates Northern blot analysis of expression of human MUC18 in different prostate cancer cell lines. Poly(A)+RNA was isolated from human melanoma cells SK-MEL-28 (SK), human melanocyte (M), and human prostate cancer cells PC-3 (PC-3), DU145 (DU), TSU-PR-1 (TSUPR1), and LNCAP (LNCAP). The size of the human MUC18 mRNA is 3.3 kb. The amount of poly(A)+RNA (2.5 to 10 µg) is indicated as a number on top of each lane.

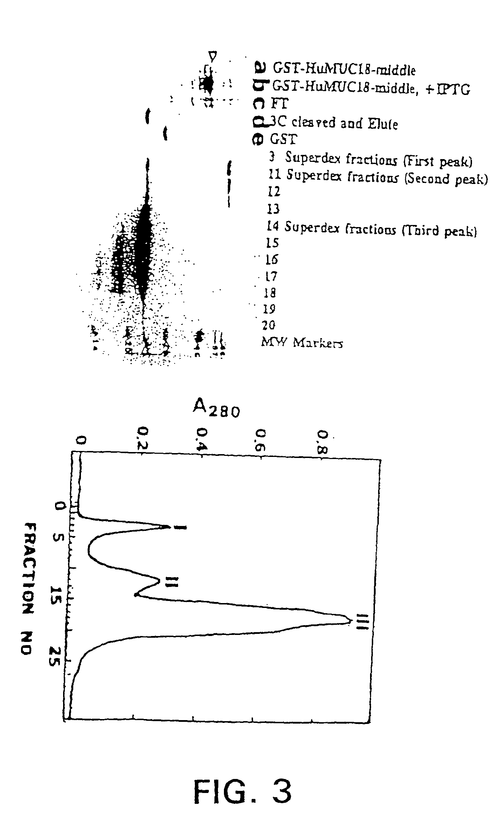

Fig. 3 depicts recombinant human MUC18-middle fragment. The plasmid map is shown in Fig. 1. The left panel shows the PAGE result and right panel the Superdex column purification. The GST-human MUC18 middle fragment fusion protein is shown after IPTG induction (a & b, indicated by a triangle on the left of the left panel). The fusion protein was first purified through a glutathione-Sepharose affinity column and then cleaved with the HRV-3C protease (left panel, lanes c-e). The affinity-purified recombinant human MUC18-middle fragment was then further purified through a Superdex column (right panel) to remove high molecular weight contaminants (peaks I and II, fractions 3-13). The final recombinant human MUC18-middle fragment protein is about 22 kDa (peak III, fractions 14-20), as indicated by a triangle on the right in the left panel.

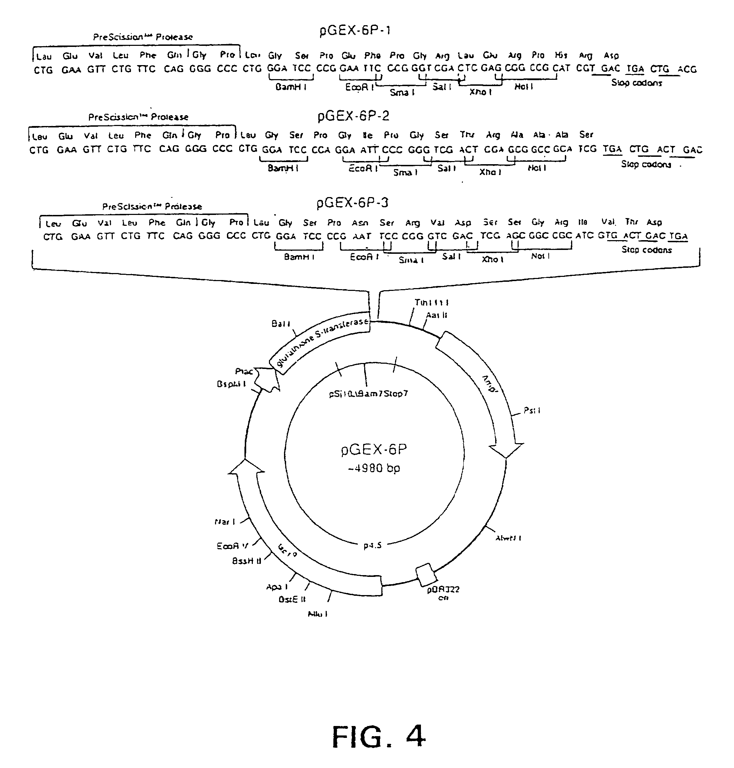

Fig. 4 shows pGEX-6P, commercially available from Pharmacia Biotech, Piscataway, NJ. The specific multiple cloning site (MCS) sequences for pGEX-6P-1 (SEQ ID NO:11, encoded amino acids, SEQ ID NO:12), pGEX-6P-2 (SEQ ID NO:13, encoded amino acids, SEQ ID NO:14) and pGEX-6P-3 (SEQ ID NO:15, encoded amino acids, SEQ ID NO:16) are provided.

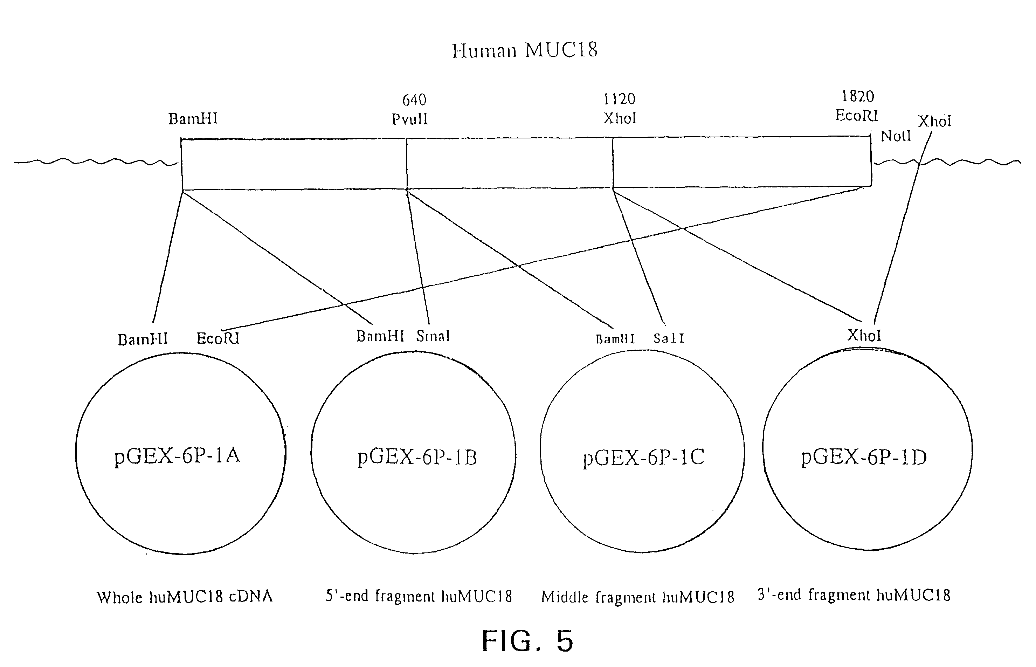

Fig. 5 provides diagrammatic illustrations of the whole cDNA sequence of human MUC18, its N-terminal fragment, middle fragment and C-terminal fragment, as cloned into the pGEX-6P-1 vector are provided. These fragments as cloned result in the expression of the N-terminal, middle and C-terminal fragments of the MUC18 protein. See also Tables 1A-1B for the locations of the relevant restriction sites in the cDNA sequence for human MUC18 and SEQ ID NO:1 for sequence.

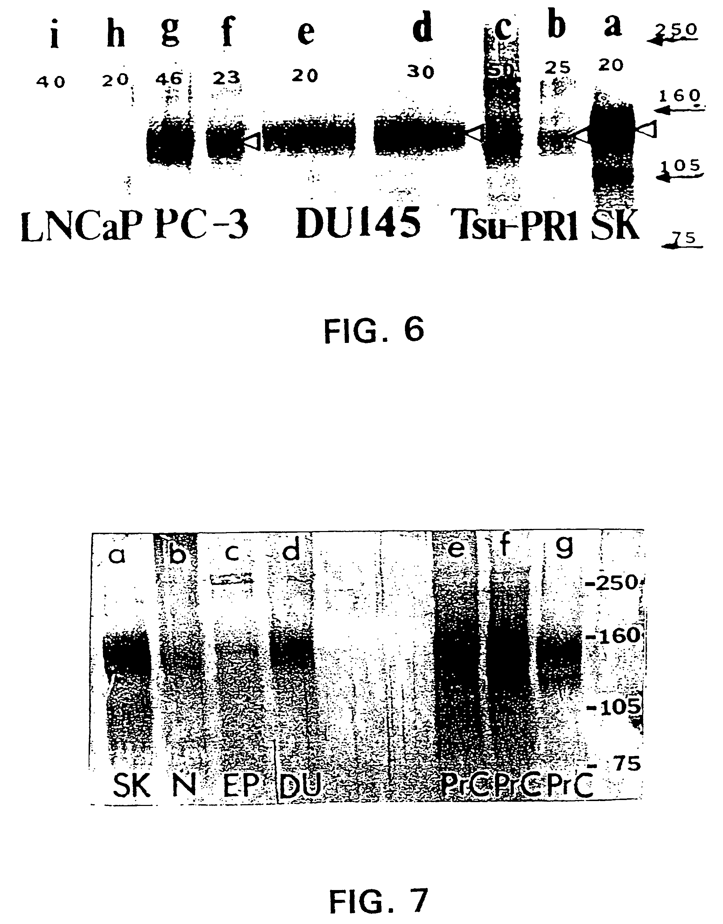

Fig. 6 illustrates the results of Western blot analysis of huMUC18 protein expression in four prostatic cancer cell lines. Cellular extracts of four prostatic cancer cell lines were prepared, and the proteins were size-separated by sodium dodecyl sulfate-polyacrylamide gel electrophoresis (SDS-PAGE). The anti-huMUC18 antibodies generated by immunization of the recombinant huMUC 18-middle portion protein in chicken were used for Western blot analysis. SK stands for cellular lysate prepared from human SK-Mel-28 cells (a), Tsu-PR1 cells (b and c), DU145 for DU145 cells (d and e), PC-3 for PC-3 cells (f and g), and LNCaP for LNCaP.FGC cells (h and i). The number over each lane indicates the amount of protein (µg) loaded in each well. The huMUC18 protein band is indicated with an open triangle. The numbers on the right-most lane indicate the protein molecular weight (kDa) rainbow markers (RPN800, Amersham).
Fig. 7 shows the results of Western blot analysis of human MUC18 protein in normal prostatic gland, normal primary human prostatic epithelial cells, and tissues of a patient with malignant prostatic cancer. Cellular lysates were prepared from normal prostatic gland (b), cultured normal primary prostatic epithelial cells (c), and prostatic cancerous tissues from a patient with malignant prostatic cancers (e-g). Cellular extracts prepared from human SK-Mel-28 cells (a) and from DU145 cells (d) were shown as the positive controls. 20µg protein of each lysate was loaded per well. The numbers on the right-most lane indicate the protein molecular weight (kDa) rainbow markers (RPN800, Amersham).

### DETAILED DESCRIPTION OF THE INVENTION

Prostate cancer accounts for about 10% of all cancer cases in the United States. It is now the most frequently diagnosed cancer in American males [Rinker-Schaeffer et al. (1993) *Cancer and Metas. Rev.* 12, 3-10]. In some patients, prostate cancers metastasize rapidly, killing the patient within one year of the initial clinical presentation. In contrast, some other prostate cancer patients show a relatively slow growth of the malignant tumor without metastasis. The majority of the histologically localized prostate cancers remain subclinical and never require treatment. Prostate cancer, when truly localized, can be cured by radical prostatectomy. While in many affected patients, the tumors are slow-growing and nonmetastatic, in others prostate cancer takes a more aggressive course. Unfortunately, metastatic prostate cancer is a fatal disease without treatment.

At present, the test for serum levels of prostate specific antigen (PSA) and the radionuclide bone scan are the only diagnostic tests available before therapy is initiated. However, an elevated level of PSA observed in serum is not predictive of the pathological state of prostate cancer, nor is it correlated with metastatic prostate cancer. This is, at least in part, because PSA, a serine protease, is not exclusively expressed in the epithelial cells of metastatic prostate cancer, but it is also expressed in normal epithelial cells, primary prostate cancerous tumors and benign prostatic hyperplasia [Wood et al. (1994) *Cancer* 74, 2533-2540]. To date, it has not been possible to predict whether histologically detected localized tumors are likely to progress to clinical cancer or when these localized tumors are likely to metastasize to other sites within the body. Thus, there is an urgent need for biochemical markers which serve to identify prostate cancers which have progressed to a stage which requires immediate surgical removal and/or additional chemotherapeutic or radiation therapeutic treatments, i.e., those cancers which are likely to metastasize.

To identify a diagnostic marker which is improved over PSA, it is crucial to understand the biochemical differences between the malignant state and the benign state and between tumor cells with high and low metastatic potential. Overexpression and underexpression of certain cell adhesion molecules at the cell surface has been proposed to reflect metastasis of several cancers [Tang and Honn (1994-1995) *Invasion Metas*. 14, 109-122]. For example, the low expression of E-cadherin has been correlated with poor prognosis of prostate cancer [Rinker-Schaeffer et al. (1993) supra]. The present invention provides a positive correlation between the level of MUC18 expression and high metastatic potential of prostate cancer cells.

The human MUC18 (huMUC18) cDNA sequence (see Table 1A) obtained by the present inventors is different from the huMUC18 cDNA sequence given in GenBank Accession No. N28882 [Johnson et al. (1994)]. The deduced amino acid sequence of huMUC18 cDNA given in Table 1A was identical to the huMUC18 sequence deposited in GenBank by Johnson's group except seven amino acid residues. This discrepancy of amino acid sequence may be due to allelic differences. However, the amino acid sequence of the inventor's huMUC18 (646 amino acids) was very different from that published by Johnson's group in 1989 (603 amino acids), which 1989 sequence appears to contain sequence errors in the huMUC18 cDNA.

The human MUC18 cDNA sequence disclosed in Table 1A was amplified by RT-PCR from poly(A)+RNA, which was isolated from the human melanoma cell line SK-Mel-28. The cDNA was cloned into the pGEM-T-easy vector (Promega, Madison, WI). The DNA sequence of the huMUC18 cDNA was determined by rapid DNA sequencing using ABI prism dye terminator cycle sequencing ready reaction kit (Perkin-Elmer) with various huMUC18 specific primers in an automated sequencer of ABI 373XL system. The Lasergen and GCG programs were used for comparison of nucleotide and amino acid sequences of the huMUC18 cDNA.

A cDNA sequence of a human MUC18 clone has been published in GenBank under Accession No. M 28882 (See Table 1B). The translation initiation codon (ATG) is underlined, as is the translation termination codon (TAG). The PvuII (CAGCTG) and XhoI (CTCGAG) restriction sites are boxed, and cut sites are indicated by vertical arrows.

Table 1C (see also SEQ ID NO:5) shows the human MUC18 sequence as modified to introduce a BamHI site (GGATCC) just upstream of the translation start site to facilitate cloning. The translation initiaition (ATG) and termination (TAG) codons are boxed. The long arrows near the 5' and

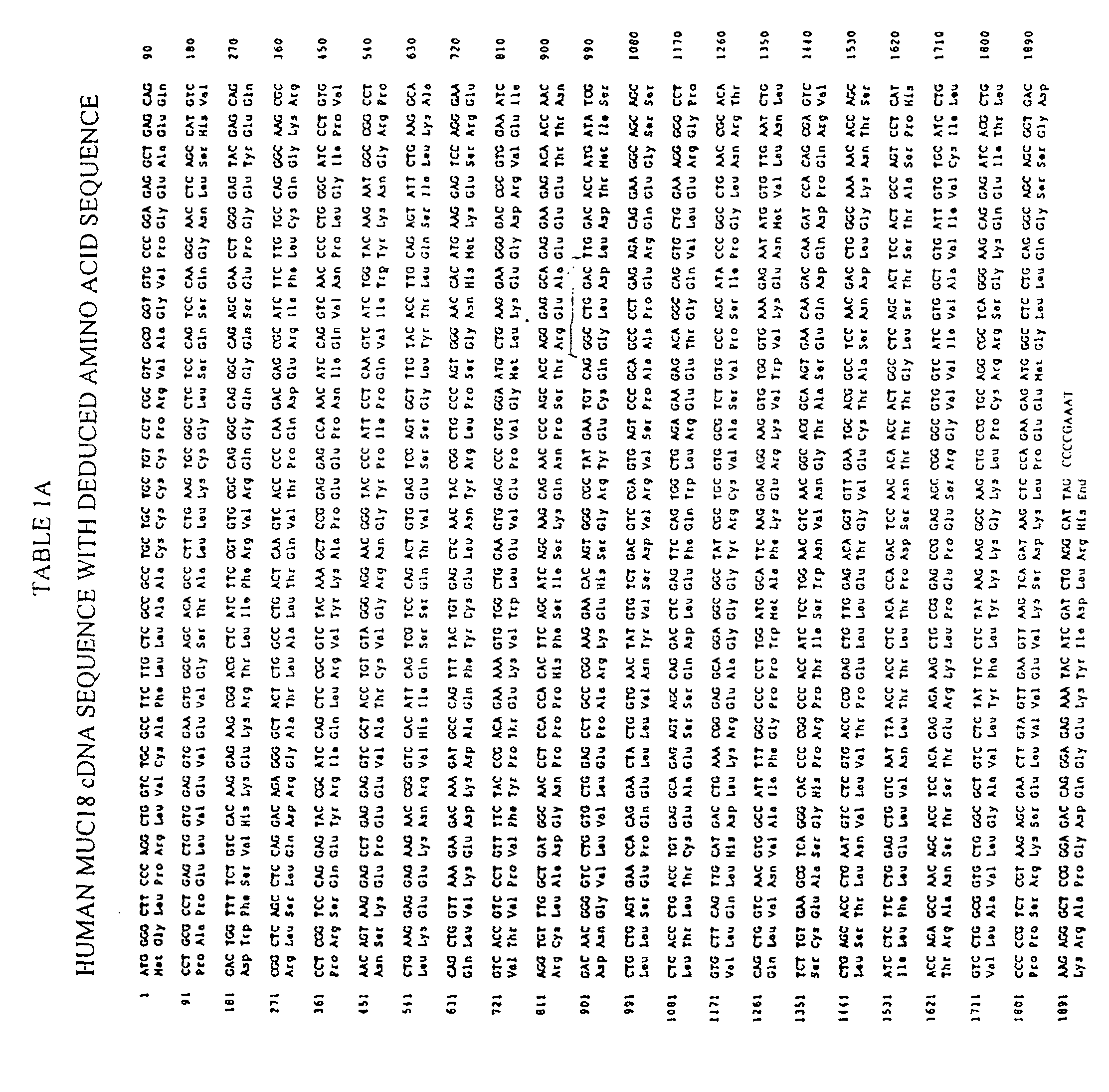

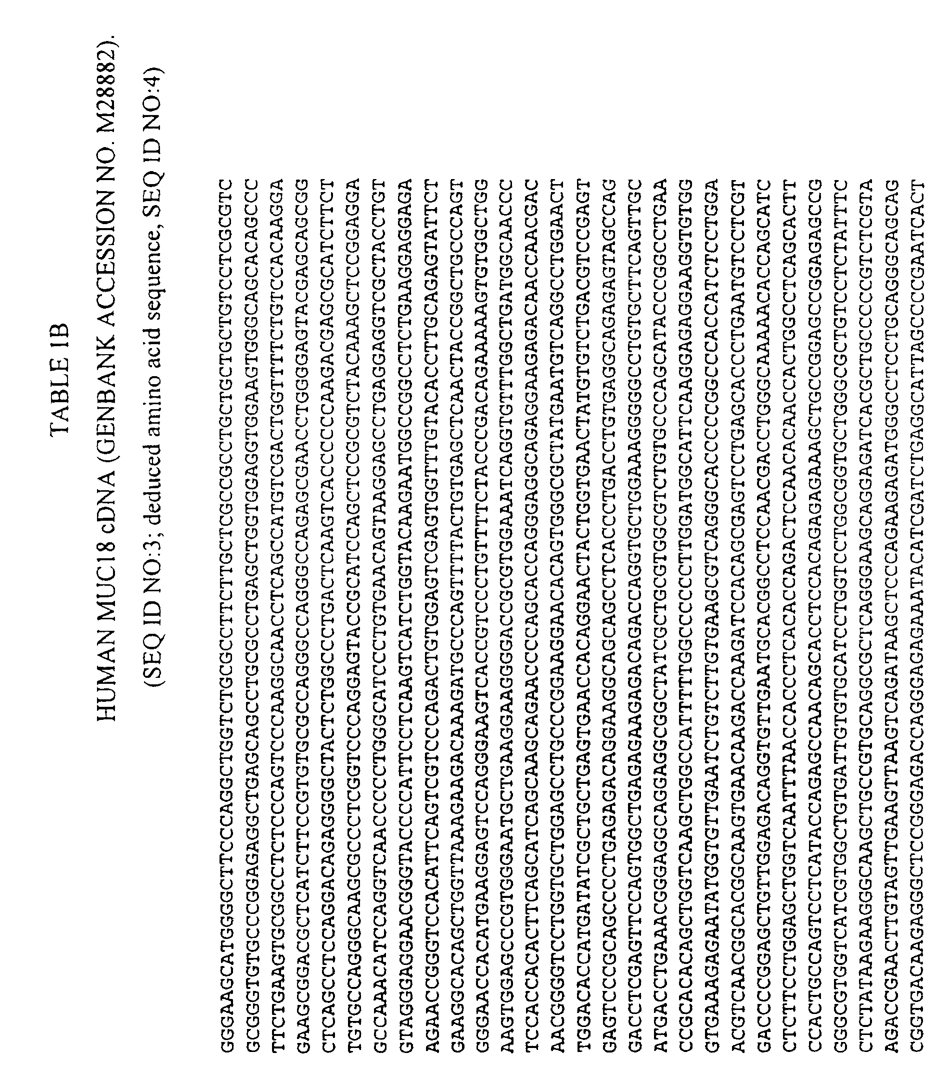

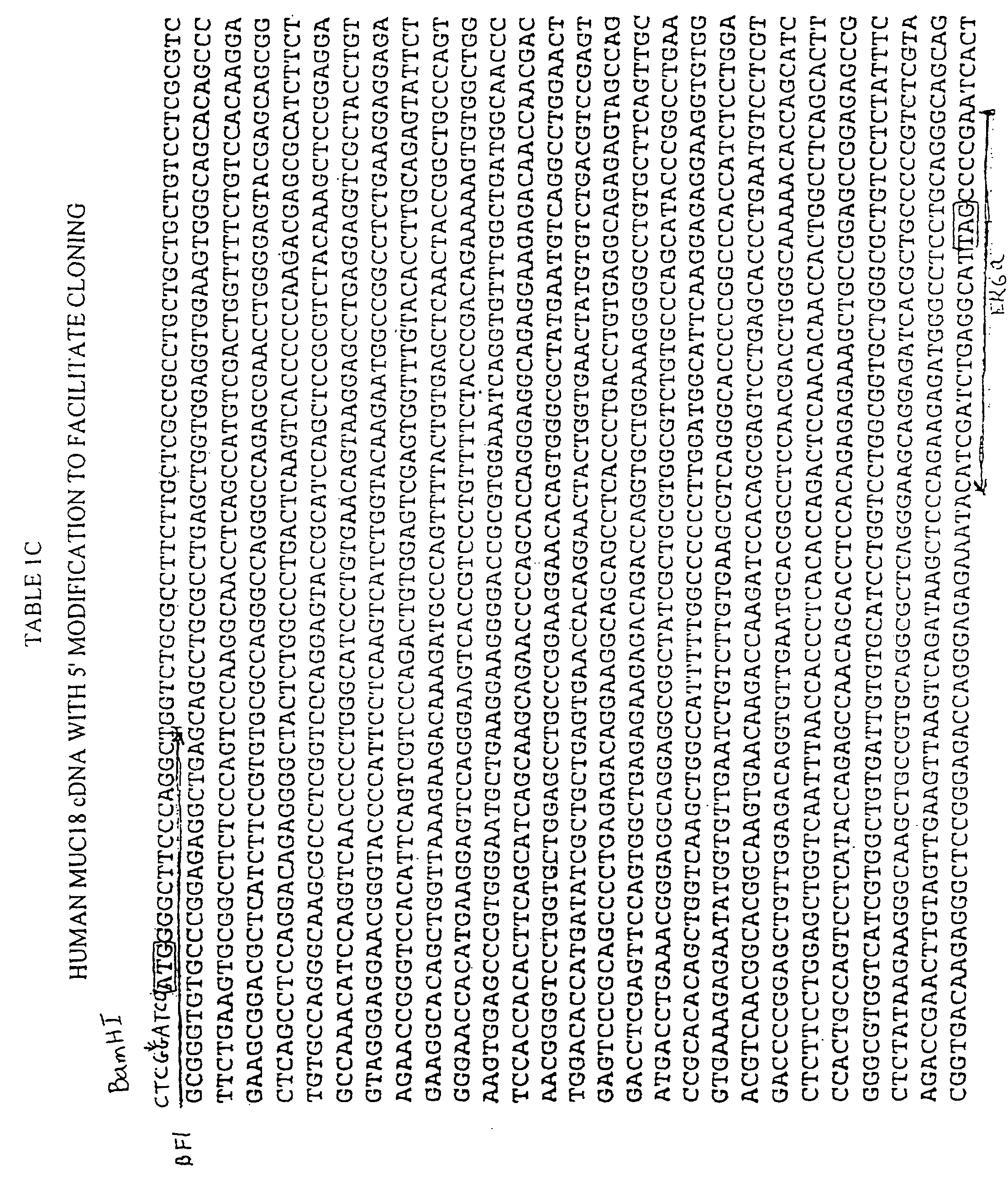

3' ends indicate primer positions (BF1 and ER6a, respectively). The cut site for BamHI within its recognition sequence (GGATCC) is indicated with a vertical arrow.

The Genbank M 28882 sequence (given in SEQ ID NO:3) is identical to a human MUC18 cDNA clone (huMUC18) from human SK-Mel-28 cells, a human malignant melanoma cell line which produces relatively high levels of the MUC18 protein. This sequence is slightly different from the huMUC18 previously published [Johnson and Rummel (1996) in *Immunology of Human Melanoma,* ed, Maio, M., IOA Press, Washington, DC, pp. 31-38; Lehmann et al. (1989) *Proc. Natl. Acad. Sci. USA* 86, 9891-9895; Luca et al. (1993) *Melanoma Res.* 3, 35-41]. The two cDNAs have three stretches of amino acid (aa) residues that are different, such as 19 aa and 17 aa at the N-terminal portion and 17 aa near the C-terminal portion. Furthermore, the published human MUC18 cDNA sequence was missing 42 amino acids at the C-terminal end (see SEQ ID NO:4). The human and murine cDNAs have 74.5% identity in the deduced amino acid sequences. The 3'-end primer used previously, ER6, did not include the last few codons and the termination codon. To re-clone the intact correct human MUC18 cDNA, a correct new 3'-primer, ER6a, for amplifying the intact human MUC 18 cDNA was designed (see hereinbelow).

Efforts to express the recombinant huMUC18 protein in the pCal-n expression system (Stratagene, La Jolla, CA) in *E. coli* failed. Finally, the expression was possible by using a GST-fusion protein expression system. The huMUC18 cDNA was cloned in the PGEX-6p-1 vector (Pharmacia), a small amount of the nearly intact MUC18 protein in *E. coli* was expressed. Fortunately, the sequence of the middle portion of the huMUC18 cDNA was correct, and it was then used for making recombinant protein in *E. coli.* Only when the middle 166 amino acid portion encoded by the cDNA, but not the N-terminal or C-terminal portions, was used for expression, over-expression of the recombinant protein was possible. One pair of primers: BamHI-HMUC18-pvuII (28-mer, GGATCCCAGCTGGTTAAAGAAGACAAAG) (SEQ ID NO:6) and HMUC18-xhoI (27-mer, CTGGAACTCGAGGTCCTGGCTACTCTC) (SEQ ID NO:7) were used for PCR-amplification of the region from PvuII to XhoI of the HuMUC18 cDNA. The amplified fragment was cloned into pGEM-T Easy vector. The DNA fragment that included the coding region from the PvuII site to the XhoI site was excised from the pGEM-T Easy recombinant plasmid by two restriction enzymes, BamHI and Sall, and cloned into the BamHI and Sall cleaved pGEX-6P-1 vector. The recombinant HuMUC18-middle fragment after cleavage with PrScission protease and purification contained the following sequences (see also Table 1A and SEQ ID NO:2).

A 22 kDa protein fragment was expressed from the PvuII to XhoI fragment of the cDNA (see Tables 1A and 1C). A large-scale preparation of the recombinant human MUC18 "middle portion" fragment was carried out. More than 6 mg of the purified recombinant protein was obtained after purification through a glutathione-affinity column, cleavage with the HRV-3C protease, being eluted and concentrated, and further purification through a Superdex column in a Pharmacia FPLC system. After final concentration of the eluant, 6 mg of the "middle portion" recombinant protein was sent to Lampire Biological Laboratories to make polyclonal antibodies in chickens. High antibody titers were reported. Eggs are collected and IgY (chicken antibody protein) is purified from these eggs. After the titers of these purified IgY preparations are determined, they are used for immunological testing.

Using the MUC 18-specific antibody preparation from chickens after immunization with the purified human recombinant huMUC18-middle fragment protein, the present inventor has shown that these antibodies can react with the human MUC 18 protein expressed in human prostate cancerous cell lines and prostatic cancerous tissues by Western blot analysis. The results showed that the human MUC 18 protein was only expressed in three metastatic prostate cancer cell lines (Tsu-PR-1, DU145 and PC-3), but not in one non-metastatic cell line (LNCaP.FGC). These results are consistent with the Northern blot analysis of the expression of huMUC18 mRNA in these prostatic cancer cell lines, described herein. The human MUC 18 protein was weakly expressed in normal prostatic epithelial cells and in normal prostate gland, but highly expressed in human cancerous prostatic tissues, as shown by Western blot analysis of the extracts prepared from these tissues. See Fig. 6 for the results of Western blot analysis.

Further immunohistochemical analysis revealed that huMUC18 is expressed in the membrane of the expected special cell types, such as metastatic melanoma tissues, endothelial cells, and smooth muscle cells. This indicates that the antibodies are very specific for huMUC18 antigen and work well with the formaldehyde-fixed, paraffin-embedded tissue sections. As expected, the antibodies did not react with any antigens of the normal secretory epithelial cells in the acini of the prostate gland. Interestingly, they react with the secretory epithelial cells in the acini of the prostate cancer tissues. The expression of human MUC18 protein (or antigen) only in cancerous epithelial cells, but not in normal epithelial cells, supports the use of human MUC18 as a diagnostic marker for the metastatic potential of prostate cancers.

Table 2 summarizes the results for MUC 18 expression in four prostate cancer cell lines in comparison with pertinent results published by other groups. Expression of MUC18 in these cell lines is consistent with their low or no expression of E-cadherin and α-catenin and their extent of invasiveness *in vitro* and metastasis in nude mice. See Fig. 6 for Western blot analysis.

The present work has correlated relatively high levels of MUC18 with the ability of prostate cancer cells to metastasize. High levels of MUC18 expression were observed in the three metastatic prostate cancer cell lines TSU-PR1, DU145 and PC-3. MUC18 expression was not detectable in the LNCAP prostate cell line, which is not metastatic. Nonmetastatic prostate cancer cells and normal prostate cells produce no or barely detectable expression of MUC18 either as protein or mRNA. Experiments in which the LNCAP cell line is genetically engineered to express MUC 18 at high levels demonstrate that when cells gain the capacity to express MUC18 at high levels, those cells gain the ability to metastasize. Experiments in which the nonmetastatic prostate cancer cell line LNCaP.FGC is genetically engineered to express MUC18 at high levels demonstrate that when the cells gain the capacity to express MUC18 at high levels, the ability to metastasize is also gained. Thus, the relative level of MUC 18 expression in prostate tumor tissue is correlated with the ability to metastasize, and measurement of MUC18 expression in prostate tumor biopsy tissue allows the medical practitioner to choose the most appropriate therapy for each prostate cancer patient, with high levels of MUC18 expression mandating an aggressive treatment strategy, likely including surgery, chemotherapy and/or radiation.

**TABLE 2**

| RELATIVE LEVELS OF MUC 18 EXPRESSION | | | | | |
|---|---|---|---|---|---|
| | TSU-PR1 | DU145 | PC-3 | LNCAP | Reference |
| E-cadherin expression | 0 | 0.1 | 0.6 | 1.1 | Morton et al. (1993) *Cancer Res*. 53,3585-3590 |
| α-catenin expression | none | none | none | yes | Morton et al. (1993) |
| Total RNA (MUC18) | yes | yes | yes | none | this work |
| tumor growth in nude mice | yes (2000) | yes (500) | Yes (1400) | yes (2000) | Passaniti et al. (1992) *International J. Cancer* 51,318-324 |
| Metastasis in nude mice | yes | yes | yes | none | Lalani et al. (1992) *Cancer Metastasis Rev.* 16,29-66 |
| Invasiveness *in vitro* | yes (220) | yes (25) | yes (20) | none | Passaniti et al. (1992) |

Monoclonal or polyclonal antibodies, preferably monoclonal, specifically reacting with MUC18, may be made by methods well known in the art. See, e.g., Harlow and Lane (1988) *Antibodies: A Laboratory Manual,* Cold Spring Harbor Laboratories; Goding (1986) *Monoclonal Antibodies: Principles and Practice*, 2d ed., Academic Press, New York. Also, recombinant immunoglobulins may be produced by methods known in the art, including but not limited to, the methods described in U.S. Patent No. 4,816,567. Monoclonal antibodies with affinities of 10⁸ M⁻¹, preferably 10⁹ to 10¹⁰ M⁻¹ or more, are preferred.

Antibodies (polyclonal or monoclonal) specific for MUC18 are useful, for example, as probes for screening DNA expression libraries or for detecting the presence (and relative amounts) of MUC18 in a test sample, for example, prostate tumor biopsy tissue or a tissue slice of a metastatic prostate cancer, or cells in culture which were derived from a primary prostate cancerous tumor or a metastatic prostate cancer tumor. Desirably, the results are normalized to cell number or to total cellular protein. Frequently, the polypeptides and antibodies are labeled by joining, either covalently or noncovalently, a substance which provides a detectable signal. Suitable labels include, but are not limited to, radionuclides, enzymes, substrates, cofactors, inhibitors, fluorescent agents, chemiluminescent agents, magnetic particles and the like. United States Patents describing the use of such labels include, but are not limited to, Nos. 3,817,837; 3,850,752; 3,939,350; 3,996,345; 4,277,437; 4,275,149; and 4,366,241.

Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al. (1989) *Molecular Cloning*, Second Edition, Cold Spring Harbor Laboratory, Plainview, New York; Maniatis et al. (1982) *Molecular Cloning,* Cold Spring Harbor Laboratory, Plainview, New York; Wu (ed.) (1993) *Meth. Enzymol*. 218, Part I; Wu (ed.) (1979) *Meth Enzymol.* 68; Wu et al. (eds.) (1983) *Meth. Enzymol.* 100 and 101; Grossman and Moldave (eds.) *Meth. Enzymol*. 65; Miller (ed.) (1972) *Experiments in Molecular Genetics,* Cold Spring Harbor Laboratory, Cold Spring Harbor, New York; Old and Primrose (1981) *Principles of Gene Manipulation*, University of California Press, Berkeley; Schleif and Wensink (1982) *Practical Methods in Molecular Biology*; Glover (ed.) (1985) *DNA Cloning* Vol. I and II, IRL Press, Oxford, UK; Hames and Higgins (eds.) (1985) *Nucleic Acid Hybridization*, IRL Press, Oxford, UK; and Setlow and Hollaender (1979) *Genetic Engineering: Principles and Methods*, Vols. 1-4, Plenum Press, New York. Abbreviations and nomenclature, where employed, are deemed standard in the field and commonly used in professional journals such as those cited herein.

The following examples are provided for illustrative purposes.

### EXAMPLES

### Example 1. Recombinant MUC18 Production and Antibody Production

The human MUC18 cDNA (1970 bp, RT-PCR amplified fragment) and three sub-fragments have been cloned in-phase in a GST-fusion protein expression system, pGEX-6p-1 (Pharmacia), which contains the glutathione-S-transferase (GST) coding region as an affinity-tag for the inserted foreign protein at its C-terminus. Fig.1 and Fig. 5 show the four possible fusions: the whole region, the N-terminal fragment, the middle fragment, and the C-terminal fragment.

Only the middle fragment of the human MUC18 protein can be induced by IPTG to express in a high amount in *E. coli* K-12 strain BL-21. Thus, only this protein is further purified for immunization. When culture A₆₀₀ reaches 0.6 (2 to 3 hours after 1/100 inoculation of an overnight culture in L-broth with ampicillin), the expression of the recombinant middle fragment of MUC18 protein fused to GST in recombinant *E. coli* is induced by addition of 0.1 mM of IPTG to 3-liter cultures (1.5 liters per 4-liter baffled flask). Two hours after addition of IPTG at 37°C, cells are harvested by centrifugation at 3,000 rpm (2,323 x g) for 20 min in a horizontal HG-4L rotor in Sorvall RC-3 centrifuge. The cell pellet is suspended in 40 ml of ice-cold PBS (10 mM Na₂HPO4, 1.8 mM KH₂PO4, 2.7 mM KCI, and 140 mM NaCl, pH 7.3) and then lysed with a prechilled French pressure cell at 800 psi. The lysate is clarified by centrifugation for two to three times at 13,000 rpm (21,000 x g) for 30 min in SS-34 rotor in Sorvall RC-2 centrifuge. The protein concentration of the clear crude lysate adjusted to 10 mg/ml protein (about 60 ml) was used as the starting material for purification. The recombinant MUC 18 proteins are purified from the clear crude lysate by batchwise adsorption to the Glutathione-Sepharose 4B affinity resin (about 20 ml of 50% slurry) by inversion on an inversion shaker at room temperature for 30 min. The GST portion of the fusion protein mediates the binding of the protein to the resin via the glutathione. After twice washing with 10 volumes (50 ml per 5 ml packed resin) of 1 X PBS and followed by twice washing with 1 X PreCission protease cleavage buffer (50 mM TrisHCl, pH 7.0, 150 mM NaCl, 1 mM EDTA, 1 mM DTT) to remove unbound proteins, the fusion protein on the resin is cleaved with 100 units of HRV-3C protease (PreCission protease, 2 units/µl, from Pharmacia) by rocking on an inversion shaker for 16 hours at 4°C. The resin was spun down at 2,000 rpm for 10 min in a Sorvall RC-32 centrifuge. The supernatant and three washings (10 ml 1 X PBS per 10 ml resin), which contain the recombinant MUC18 protein, are then combined and concentrated by centrifuging through a Centricon-30 (Millipore/Amicon). The purity of the protein is characterized by SDS-PAGE (8 to 10% polyacrylamide gel, slab gel). The 70kDa contaminated protein is removed by passing through a Superdex 200 HR 10/30 column in 1 X PBS (void volume about 7 ml for a 20 ml packed column), and the fractions containing the recombinant middle fragment MUC18 protein (22 kDa) (eluted at about 15.5 ml) were pooled. Fig. 2 shows the SDS-PAGE results or recombinant huMUC18 protein in the GST-fusion system.

Six mg of protein is sent to Lampire Biological Lab. (Pipersville, PA) for immunizing three chickens. The anti-MUC18 antibody titers are determined by ELISA assay using the purified recombinant MUC18 as the antigen. Eggs collected during the period of high serum antibody titers are used for purification of chicken immunoglobulin IgY.

To confirm the association between MUC18 expression and metastatic ability of prostate cancer cells, the human MUC18 coding sequence is introduced into a non-metastatic, non-expressing human prostate cancer cell line (LNCAP), and clones with different levels of expression of MUC18 are isolated.

The human MUC18 cDNA has been cloned into pCR3.1 Uni (Promega, Madison, WI), a mammalian expression vector in which high levels of gene expression are driven by the human CMV-IE promoter. The human MUC18 cDNA is also cloned into a murine amphitrophic retrovirus expression vector, e.g. pZipNeoSVX [Cepko et al. (1984) *Cell* 37, 1053-1062] or LXSN [Miller and Rosman (1989) *BioTechniques* 7, 980-990], in which LTR drives gene expression.

These MUC18 recombinant vectors are used to transfect a human prostate cancer cell line which does not express MUC18, for example the LNCaP.FGC cell line [Umbas et al. (1992) *Cancer Res.* 52, 5104-5109; lizumi et al (1987) *J. Urol.* 137: 1304-1306]. The vectors are introduced into the cultured cells by lipofection [Felgner et al. (1987) *Proc. Natl. Acad. Sci. USA* 84, 7413-7417] or by electropporation [Potter, H. (1988) *Anal. Biochem.* 174:361-373]. G418-resistant clones are selected and purified [Yuo et al. (1992) *Intervirol*. 34, 94-104] in view of the kanamycin resistance coding sequence expressed under the control of the SV40 promoter in each of the vectors. Relative expression levels of MUC18 expression in different clones are determined by western blotting with polyclonal antibodies (described herein). Metastatic abilities are determined as described herein.

### Example 2. Determination of Metastatic Ability of Prostate Cancer Cells

The degree of motility and the invasiveness of prostate cancer cells are quantitated using published methods [Tucker et al. (1994) *Eur. J, Cell Biol.* 58, 28-290; Repesh, L.A. (1989) *Invasion and Metastasis* 9, 192-208]. The Costar transwell chamber contains an inner well with a porous polycarbonate membrane, with 3 µm pore sizes in the bottom of the well. This is tightly fitted to the outer well.

To determine the motility of human prostate cancer cells, 0.5 x 10⁵ cells are seeded in the top well. The cells remain on the top well because the poly carbonate membrane only allows medium to pass through freely. After seeding and attachment, the cells in the top well gradually migrate through the pores in the polycarbonate membrane to the bottom side of the membrane. Eventually some cells establish growth at the bottom side of the membrane. When the pore size of the membrane is about 3 µm, it somewhat slows the movement of the cells from the top side of the membrane to the bottom side. Motility of the cells is measured over the next several consecutive days. The rate of motility of a given cell line can be determined quantitatively by counting the cell number at the bottom of the membrane after trypsinization. Using this in vitro method, the motility rates of PC-3 and PC-huMUC18 human prostate cancer cells, with and without an over-expression of the human MUC 18 protein, respectively, are determined and compared.

For the invasiveness of the prostate cancer cells, a similar kind of chamber is used, except before seeding the cells to the top well, the polycarbonate membrane is pre-coated with matrigel that contains protein components of the basal membranes of blood vessels. When the concentration of matrigel is correct, the membrane thus formed is thick enough to form a barrier to stop the cells from penetrating immediately, but is thin enough to allow cells to gradually invade through the membrane and migrate to the bottom of the membrane. Matrigel, which contains protein components of the base membrane of blood cell membrane, such as laminin, collagen type IV, entactin (nedogen), and heparin sulfate proteoglycan, is available commercially through Collaborative Research (Bedford, MA). Each filter in each 6.5 mm well is coated with 100 µl of a 1:20 dilution of commercial Matrigel in cold DMEM (about 30 µg per filter). Using a similar method of counting the cells, which grow and attach to the bottom of the membrane, the rate of invasiveness of a given cell line can be quantitated [Repesh, L.A. (1989) supra]. To count the cells at the bottom of the membrane, the bottom of the membrane is treated with trypsin to detach the cells, and the cell number is counted directly using a Hemacytometer, or the cells on the membrane is stained with trypan blue and counted directly using a microscope. Alternatively, the cells are labeled with 0.6 µCi of ¹²⁵I-iododeoxyuridine (5 Ci/mg) for 18-24 h (about 95% of cells) and seeded to the top of the transwell chamber. After 72 h of invasion, the cells at the bottom and the cells on the top are trypsinized. The total input radioactivity is determined and compared to the radioactivities associated with cells from the top and bottom chambers. In this way the percentage of cells invading through the membrane can be accurately quantitated. Invasion rate can thus be determined [Repesh, L.A. (1989) *Invasion & Metast*. 9:192-208]. This type of in vitro test has been demonstrated to produce results in agreement with that of the in vivo animal tests [Repesh, L.A. (1989) supra]. Using this in vitro method, the invasion rates of LNCAP and LNCAP-huMUC18 transformed or transfected to express MUC18 human prostate cells, with and without over-expression of the human MUC18 protein, respectively, are determined and compared.

For comparison, the metastasis rates of these prostate cells are also tested in vivo in athymic nude mice [van Weerden et al. (1996) *Am. J. Pathol*. 149, 1055-1062]. The effect of the different expression levels of human MUC18 in prostate cancer cells on metastatic ability of different clones is determined. Similar pairs of cells as used for in vitro assay are also used in animal tests. The cells are implanted subcutaneously into nude mice. The size of tumors after different times are measured. The time and extent of the tumor cell metastases to bone or to other organs are investigated [van Weerden et al. (1996) supra].

The experiments set forth above confirm that the relative level of MUC18 expression in prostate cancer cells correlates positively with metastatic ability.

### Example 3. Transcriptional Expression of MUC18

Total cell RNA is prepared from cell lines or from prostate tumor tissue with the method of one step acid-guanidinium-thiocyanate-phenol-chloroform extraction [Chomczyuski and Sacchi (1987) *Anal. Biochem*. 162, 156-159]. We have shown that this method consistently yields good quality RNA for RT-PCR. Poly(A)+RNA is prepared from total RNA by purifying through an oligo(dT)-cellulose column [Aviv and Leder (1972) *J. Molec. Biol*. 134, 743; Wu et al. (1985) *Int. J. Biochem.* 17, 355-363].

Northern hybridization technology is used to determine MUC18 mRNA levels expressed in the four prostate cancer cell lines [Ausubel et al. (1987) *Current Protocols in Molecular Biology,* Section 4]. Total RNA is extracted from cell preparations, electrophoretically separated in a formaldehyde-impregnated agarose gel, blotted and hybridized to a ³²P-labelled human MUC18 cDNA [Feinberg and Vogelstein (1983) *Anal. Biochem*. 132, 6-13]. Relative levels of MUC18 mRNA are detected by radioautography. The amount of RNA loaded and transferred is estimated by the intensities of the 28S and 16S bands in each lane. Alternately, G3PDH mRNA can be estimated using a G3PDH-specific probe or 28S and 18S probes after stripping the MUC18-specific probe.

Quantitative RT-PCR methodology [Innis et al. (1990) PCR Protocols, Academic Press; Quantitative RT-PCR (1993) Methods and Applications Book #3, Clontech, Palo Alto, CA]. Human Sk-Mel-28 cells are used as positive control for this method.

Because the quantity of mRNA from a small amount of tissue is small, quantitative RT-PCR is used for quantifying MUC18 mRNA expression in tissues [Innis et al. (1990) supra]. The isolation kit from Boehringer-Mannheim (Indianapolis, IN) using magnetic beads is suitable for obtaining a small amount of poly(A)+RNA from prostate tissue. The quality of mRNA isolated with this kit is also excellent for translation and RT-PCR. The quantitative RT-PCR method is first established from using mRNA of cultured cells, as described above, and then is used for quantifying the expression of MUC18 mRNA in different prostate cancer tissues.

The RT step is standard: a 20 µl RT reaction contains 1 µg of poly(A)+RNA from the human melanoma cell line, SK-Mel-28, as template, 0.5 µg of oligo(dT)₁₆ as primer (Promega), 2 µl of 10X AMV-RT buffer (Promega, Madison, WI), 2 units of AMV-reverse transcriptase, 5 mM MgCl₂, 1 mM of dNTP mix (Promega), 1 unit of RNase inhibitor (Promega), and 50 µg/ml of acetyl BSA. The reaction was carried out at 42 to 48°C for one hour, and heated at 99°C for 5 min.

A 20 µl PCR reaction contained 2 µl of RT reaction mixture (containing the first-strain cDNA), 2 µl each of the two primers (20 pm/µl), 0.01 mM dNTPs, 1 µg of acetyl BSA, 2 µl of 10X PCR buffer with 15 mM MgCl₂ (Promega), and 0.5 units of Taq DNA polymerase (Promega, 5 units/µl). PCR cycles are as follows:
Hot start at 94°C 5 min, 80°C 30 sec
29 cycles of 94°C 30 sec, 64-66°C 30 sec, and 72°C 2 min
1 cycle of 94°C 30 sec, 64-66°C 30 sec, and 72°C 60 min

The sequences of the primers for amplification of the human MUC18 cDNA from poly(A)+RNA of human melanoma cell line SK-Mel-28 are as follows:

### Example 4. Immunofluorescence Assay for MUC18

Where the anti-human MUC 18 antibodies are made in chicken, fluorescence-tagged anti-chicken IgG are used for immunofluorescent staining. Tissue culture cells, or normal or cancer prostate tissue samples are fixed, and first reacted with the anti-human MUC18 antibodies, washed, and then reacted with the fluorescence-tagged rabbit anti-chicken antibodies [Umbas et al. (1992) *Canc. Res.* 52, 5104-5109]. The presence of human MUC18 on the surface of these cells or tissues readily detected using UV-fluorescence microscopy.

Human melanoma cells, Sk-Mel-28 (ATCC HTB 72), which express MUC18 a high level of MUC18 are used as the positive control. Human melanoma cells, WM115 (ATCC CRL 1675) which express no MUC 18, are used as the negative control. Three human prostate cancer cell lines, LNCap.FGC, PC-3, and DU145 are available from American Type Culture Collection, Rockville, MD, as ATCC CRL 1740, ATCC CRL 1435 and ATCC HTB 81, respectively. One other cell line, TSU-PR1, isolated by Dr. Iizumi in Japan [Iizumi et al. (1987) *J. Urol.* 137, *J. Urol*. 137:1304-1306] and provided by Dr. John T. Isaacs, John Hopkins University, Baltimore, MD] was tested. TSU-PR1 cells are more metastatic than the other three cell lines [Graff et al. (1995) *Cancer Res.* 55, 5195-5199].

All four cell lines are grown as monolayer cultures in a 37°C incubator with an atmosphere of 5% CO₂. Tsu-PR1 and LNCap.FGC cells are grown in RPMI 1640 supplemented with 10% fetal bovine serum. PC-cells are grown in F12K medium with 10% fetal bovine serum. DU145 cells are grown in EMEM medium supplemented with pyruvate, extra non-essential amino acids and vitamins, and 10% fetal bovine serum.

Biopsy samples are taken, fixed and subjected to immunoassay using polyclonal antibody specific for human MUC18 described hereinabove, as described in Wood et al. (1994) *Cancer* 74:2533-2540.

## Claims

1. An in vitro method for identifying metastatic potential of a prostate cancer cell, said method comprising the step of :
detecting expression of a MUC 18 coding sequence in a prostate cancer cell for which an identification of metastatic potential is sought relative to expression of a MUC 18 coding sequence in a normal prostate cell, wherein a higher level of expression of the MUC18 coding sequence is positively correlated with metastatic potential of a prostate cancer cell,
whereby metastatic potential of a prostate cancer cell is deemed high when the level of expression of a MUC 18 coding sequence is higher in said prostate cancer cell than in a normal prostate cell.

2. The method of claim 1, wherein said prostate cancer cell is from a biopsy tissue sample from a patient for whom a prediction of metastasis of prostate cancer is sought.

3. The method of claim 1, wherein expression of MUC 18 coding sequence is determined by immunoassay.

4. The method of claim 3, wherein expression of the MUC18 coding sequence is determined by immunoassay using antibody made in an experimental laboratory animal in response to a MUC 18 antigen.

5. The method of claim 4, wherein the MUC 18 antigen is a middle portion of MUC 18.

6. The method of claim 5, wherein said middle portion of MUC 18 has an amino acid sequence as given in SEQ ID NO: 2, amino acids 211-376.

7. The method of claim 1, wherein expression of a MUC 18 coding sequence is determined by Northern hybridization.

8. The method of claim 7, wherein a probe used in Northern hybridization comprises at least 15 contiguous nucleotides of SEQ ID NO: 1.

9. The method of claim 8, wherein a probe used in Northern hybridization comprises a nucleotide sequence as given in SEQ ID NO: 6, SEQ ID NO: 7, SEQ ID NO: 9, or SEQ ID NO: 10.

10. The method of claim 1, wherein said expression of a MUC18 coding sequence is determined by a reverse transcriptase-polymerase chain reaction.

11. The method of claim 10, wherein a primer used in the reverse-transcriptase polymerase chain reaction comprises a nucleotide sequence as given in SEQ ID NO: 6, SEQ ID NO: 7, SEQ ID NO: 9 or SEQ ID NO: 10.

12. The method of claim 1, wherein said prostate cancer cell is a cell line cell.

13. An immunoassay kit for diagnosing metastatic potential of a prostate cancer cell, said kit comprising an antibody made in response to immunization with an antigen consisting essentially an amino acid sequence as given in SEQ ID NO: 2, amino acids 211-376.

14. A nucleic acid vector comprising a nucleotide sequence encoding a middle portion MUC 18 protein, said middle portion MUC 18 protein consisting essentially of an amino acid sequence as given in SEQ ID NO: 2, amino acids 211-376.

15. The nucleic acid vector of claim 14, wherein said vector comprises a nucleotide sequence encoding a middle portion MUC18 protein as given in SEQ ID NO: 1, nucleotides 631-1128.

16. A recombinant host cell comprising the nucleic acid vector of claim 14.

17. A nucleic acid vector comprising a nucleotide sequence encoding a MUC 18 protein, said MUC 18 protein being **characterized by** an amino acid sequence as given in SEQ ID NO: 2.

18. The nucleic acid vector of claim 17, wherein said nucleotide sequence encoding a MUC18 protein is as given in SEQ ID NO: 1, nucleotides 1-1938.

19. A recombinant host cell comprising the nucleic acid vector of claim 17.

## Patentansprüche

1. *in-vitro*-Verfahren zum Identifizieren von metastatischem Potenzial einer Prostatakrebszelle, wobei das Verfahren die Schritte umfasst von :
Detektieren der Expression einer MUC18 kodierenden Sequenz in einer Prostatakrebszelle, für welche eine Identifikation von metastatischem Potenzial gesucht wird bezüglich der Expression einer MUC18 kodierenden Sequenz in einer normalen Prostatazelle, worin ein höherer Gehalt an Expression der MUC18 kodierenden Sequenz positiv korreliert wird mit dem metastatischen Potenzial einer Prostatakrebszelle,
wobei das metastatische Potenzial einer Prostatakrebszelle als höher vermutet wird, wenn der Gehalt von Expression einer MUC18 kodierenden Sequenz höher in der Prostatakrebszelle ist als in einer normalen Prostatazelle.

2. Verfahren nach Anspruch 1, worin die Prostatakrebszelle aus einer Biopsiegewebeprobe von einem Patienten stammt, für welchen eine Vorhersage von Metastase von Prostatakrebs gewünscht wird.

3. Verfahren nach Anspruch 1, worin die Expression von MUC18 kodierender Sequenz durch Immunotest bestimmt wird.

4. Verfahren nach Anspruch 3, worin die Expression von MUC18 kodierender Sequenz durch Immunotest unter Verwendung von einem Antikörper bestimmt wird, gemacht in einem Experimentallabortier in Erwiderung auf ein MUC18-Antigen.

5. Verfahren nach Anspruch 4, worin das MUC18-Antigen der Mittelteil von MUC18 ist.

6. Verfahren nach Anspruch 5, worin der Mittelteil von MUC18 eine Aminosäuresequenz wie in SEQ ID No: 2, Aminosäuren 211-376 hat.

7. Verfahren nach Anspruch 1, worin die Expression von MUC18 kodierender Sequenz durch Northern-Hybridisierung bestimmt wird.

8. Verfahren nach Anspruch 7, worin eine Probe, die bei Northern-Hybridisierung verwendet wird, wenigstens 15 aneinanderliegende Nukleotide von SEQ ID No: 1 umfasst.

9. Verfahren nach Anspruch 8, wobei die bei Northern-Hybridisierung verwendete Probe eine Nukleotidsequenz umfasst, die wie in SEQ ID No: 6, SEQ ID No: 7, SEQ ID No: 9 oder SEQ ID No: 10 angegeben ist.

10. Verfahren nach Anspruch 1, worin die Expression einer MUC18 kodierenden Sequenz bestimmt wird durch reverse Transkriptasepolymerase-Kettenreaktion.

11. Verfahren nach Anspruch 10, worin ein bei der reversen Transkriptasepolymerase-Kettenreaktion verwendeter Primer bzw. Starter eine Nukleotidsequenz umfasst wie angegeben in SEQ ID No: 6, SEQ ID No: 7, SEQ ID No: 9 oder SEQ ID No: 10 umfasst.

12. Verfahren nach Anspruch 1, worin die Prostatakrebszelle eine Zelllinienzelle ist.

13. Immunotestkit zum Diagnostizieren von metastatischem Potenzial einer Prostatakrebszelle, wobei der Kit umfasst einen Antikörper, der in Erwiderung auf Immunisierung mit einem Antigen gemacht ist, bestehend im Wesentlichen aus einer Aminosäuresequenz wie angegeben in SEQ ID No: 2, Aminosäuren 211-376.

14. Nukleinsäurevektor, umfassend eine Nukleinsäuresequenz, die einen Mittelteil von MUC18-Protein kodiert, wobei der Mittelteil von MUC18-Protein im Wesentlichen aus einer Aminosäuresequenz besteht wie angegeben in SEQ ID No: 2, Aminosäuren 211-376.

15. Nukleinsäurevektor nach Anspruch 14, worin der Vektor eine Nukleotidsequenz umfasst, die einen Mittelteil von MUC18-Protein kodiert wie angegeben in SEQ ID No: 1, Nukleotide 631-1128.

16. Rekombinante Wirtszelle, umfassend den Nukleinsäurevektor nach Anspruch 14.

17. Nukleinsäurevektor, umfassend eine Nukleotidsequenz, kodierend für ein MUC18-Protein, wobei das MUC18-Protein **gekennzeichnet ist durch** eine Aminosäuresequenz wie angegeben in SEQ ID No: 2.

18. Nukleinsäurevektor nach Anspruch 17, worin die Nukleotidsequenz, die ein MUC18-Protein kodiert, wie in SEQ ID No: 1, Nukleotide 1-1938 angegeben ist.

19. Rekombinante Wirtszelle, die einen Nukleinsäurevektor nach Anspruch 17 umfasst.

## Revendications

1. Procédé *in vitro* d'identification du potentiel métastatique d'une cellule prostatique cancéreuse, ledit procédé comprenant les étapes consistant à :
détecter l'expression d'une séquence codante de MUC 18 dans une cellule prostatique cancéreuse pour laquelle on chercher à identifier le potentiel métastatique par rapport à l'expression d'une séquence codante de MUC 18 dans une cellule prostatique normale, dans lequel un niveau d'expression plus élevé de la séquence codante de MUC 18 est positivement corrélé au potentiel métastatique de la cellule prostatique cancéreuse,
moyennant lequel le potentiel métastatique d'une cellule prostatique cancéreuse est considéré élevé lorsque le niveau d'expression d'une séquence codante de MUC 18 est plus élevé dans ladite cellule prostatique cancéreuse que dans une cellule prostatique normale.

2. Procédé selon la revendication 1, dans lequel ladite cellule prostatique cancéreuse provient d'un échantillon de tissu biopsique d'un patient pour lequel on cherche à établir un diagnostic de métastase du cancer de la prostate.

3. Procédé selon la revendication 1, dans lequel l'expression d'une séquence codante de MUC 18 est déterminée par un dosage immunologique.

4. Procédé selon la revendication 3, dans lequel l'expression de la séquence codante de MUC 18 est déterminée par un dosage immunologique utilisant un anticorps développé chez un animal expérimental de laboratoire en réponse à un antigène MUC 18.

5. Procédé selon la revendication 4, dans lequel l'antigène MUC 18 est une partie intermédiaire de MUC 18.

6. Procédé selon la revendication 5, dans lequel ladite partie intermédiaire de MUC 18 a une séquence d'acides aminés telle que proposée dans SEQ ID N° : 2, acides aminés 211 à 376.

7. Procédé selon la revendication 1, dans lequel l'expression d'une séquence codante de MUC 18 est déterminée par une hybridation Northern.

8. Procédé selon la revendication 7, dans lequel une sonde utilisée dans une hybridation Northern comprend au moins 15 nucléotides contigus de SEQ ID N° : 1.

9. Procédé selon la revendication 8, dans lequel une sonde utilisée dans une hybridation Northern comprend une séquence nucléotidique telle que proposée dans SEQ ID N° : 6, SEQ ID N° : 7, SEQ ID N° : 9 ou SEQ ID N° : 10.

10. Procédé selon la revendication 1, dans lequel ladite expression d'une séquence codante de MUC 18 est déterminée par une réaction d'amplification en chaîne par polymérase avec transcriptase inverse (RT-PCR).

11. Procédé selon la revendication 10, dans lequel une amorce utilisée dans la RT-PCR comprend une séquence nucléotidique telle que proposée dans SEQ ID N° : 6, SEQ ID N° : 7, SEQ ID N° : 9 ou SEQ ID N° : 10.

12. Procédé selon la revendication 1, dans lequel ladite cellule prostatique cancéreuse est une cellule d'une lignée cellulaire.

13. Kit de dosage immunologique destiné à diagnostiquer le potentiel métastatique d'une cellule prostatique cancéreuse, ledit kit comprenant un anticorps développé en réponse à une immunisation avec un antigène comprenant essentiellement une séquence d'acides aminés telle que proposée dans SEQ ID N° : 2, acides aminés 211-376.

14. Vecteur d'acide nucléique comprenant une séquence nucléotidique codant pour une partie intermédiaire d'une protéine MUC 18, ladite partie intermédiaire de la protéine MUC 18 se composant essentiellement d'une séquence d'acides aminés telle que proposée dans SEQ ID N° : 2, acides aminés 211 à 376.

15. Vecteur d'acide nucléique selon la revendication 14, dans lequel ledit vecteur comprend une séquence nucléotidique codant pour une partie intermédiaire d'une protéine MUC 18 telle que proposée dans SEQ ID N° : 1, nucléotides 631 à 1128.

16. Cellule hôte recombinante comprenant le vecteur d'acide nucléique selon la revendication 14.

17. Vecteur d'acide nucléique comprenant une séquence nucléotidique codant pour une protéine MUC 18, ladite protéine MUC 18 étant **caractérisée par** une séquence d'acides aminés telle que proposée dans SEQ ID N° : 2.

18. Vecteur d'acide nucléique selon la revendication 17, dans lequel ladite séquence nucléotidique codant pour une protéine MUC 18 est telle que proposée dans SEQ ID N° : 1, nucléotides 1 à 1938.

19. Cellule hôte recombinante comprenant le vecteur d'acide nucléique selon la revendication 17.


### SEQUENCE LISTING

<110> Wu, Guang-Jer
   EMORY UNIVERSITY
<120> Diagnostic for Metastatic Prostate Cancer
<130> 95-97 wo
<140> WO unassigned
   <141> 1999-03-02
<150> US 60/076,664
   <151> 1998-03-03
<160> 16
<170> PatentIn Ver. 2.0
<210> 1
   <211> 1950
   <212> DNA
   <213> Homo sapiens
<220>
   <221> CDS
   <222> (1)..(1938)
<400> 1
   atg ggg ctt ccc agg ctg gtc tgc gcc ttc ttg ctc gcc gcc tgc tgc   48
   Met Gly Leu Pro Arg Leu Val Cys Ala Phe Leu Leu Ala Ala Cys Cys
     1               5                  10                  15
   tgc tgt cct cgc gtc gcg ggt gtg ccc gga gag gct gag cag cct gcg   96
   Cys Cys Pro Arg Val Ala Gly Val Pro Gly Glu Ala Glu Gln Pro Ala
                20                  25                  30
   cct gag ctg gtg gag gtg gaa gtg ggc agc aca gcc ctt ctg aag tgc 144
   Pro Glu Leu Val Glu Val Glu Val Gly Ser Thr Ala Leu Leu Lys Cys
   35 40 45
   ggc ctc tcc cag tcc caa ggc aac ctc agc cat gtc gac tgg ttt tct 192
   Gly Leu Ser Gln Ser Gln Gly Asn Leu Ser His Val Asp Trp Phe Ser
   50 55 60
   gtc cac aag gag aag cgg acg ctc atc ttc cgt gtg cgc cag ggc cag 240
   Val His Lys Glu Lys Arg Thr Leu Ile Phe Arg Val Arg Gln Gly Gln
   65 70 75 80
   ggc cag agc gaa cct ggg gag tac gag cag cgg ctc agc ctc cag gac 288
   Gly Gln Ser Glu Pro Gly Glu Tyr Glu Gln Arg Leu Ser Leu Gln Asp
   85 90 95
   aga ggg gct act ctg gcc ctg act caa gtc acc ccc caa gac gag cgc 336
   Arg Gly Ala Thr Leu Ala Leu Thr Gln Val Thr Pro Gln Asp Glu Arg
   100 105 110
   atc ttc ttg tgc cag ggc aag cgc cct cgg tcc cag gag tac cgc atc 384
   Ile Phe Leu Cys Gln Gly Lys Arg Pro Arg Ser Gln Glu Tyr Arg Ile
   115 120 125
   cag ctc cgc gtc tac aaa gct ccg gag gag cca aac atc cag gtc aac 432
   Gln Leu Arg Val Tyr Lys Ala Pro Glu Glu Pro Asn Ile Gln Val Asn
   130 135 140
   ccc ctg ggc atc cct gtg aac agt aag gag cct gag gag gtc gct acc 480
   Pro Leu Gly Ile Pro Val Asn Ser Lys Glu Pro Glu Glu Val Ala Thr
   145 150 155 160
   tgt gta ggg agg aac ggg tac ccc att cct caa gtc atc tgg tac aag 528
   Cys Val Gly Arg Asn Gly Tyr Pro Ile Pro Gln Val Ile Trp Tyr Lys
   165 170 175
   aat ggc cgg cct ctg aag gag gag aag aac cgg gtc cac att cag tcg 576
   Asn Gly Arg Pro Leu Lys Glu Glu Lys Asn Arg Val His Ile Gln Ser
   180 185 190
   tcc cag act gtg gag tcg agt ggt ttg tac acc ttg cag agt att ctg 624
   Ser Gln Thr Val Glu Ser Ser Gly Leu Tyr Thr Leu Gln Ser Ile Leu
   195 200 205
   aag gca cag ctg gtt aaa gaa gac aaa gat gcc cag ttt tac tgt gag 672
   Lys Ala Gln Leu Val Lys Glu Asp Lys Asp Ala Gln Phe Tyr Cys Glu
   210 215 220
   ctc aac tac cgg ctg ccc agt ggg aac cac atg aag gag tcc agg gaa 720
   Leu Asn Tyr Arg Leu Pro Ser Gly Asn His Met Lys Glu Ser Arg Glu
   225 230 235 240
   gtc acc gtc cct gtt ttc tac ccg aca gaa aaa gtg tgg ctg gaa gtg 768
   Val Thr Val Pro Val Phe Tyr Pro Thr Glu Lys Val Trp Leu Glu Val
   245 250 255
   gag ccc gtg gga atg ctg aag gaa ggg gac cgc gtg gaa atc agg tgt 816
   Glu Pro Val Gly Met Leu Lys Glu Gly Asp Arg Val Glu Ile Arg Cys
   260 265 270
   ttg gct gat ggc aac cct cca cca cac ttc agc atc agc aag cag aac    864
   Leu Ala Asp Gly Asn Pro Pro Pro His Phe Ser Ile Ser Lys Gln Asn
           275                 280                 285
   ccc agc acc agg gag gca gag gaa gag aca acc aac gac aac ggg gtc    912
   Pro Ser Thr Arg Glu Ala Glu Glu Glu Thr Thr Asn Asp Asn Gly Val
       290                 295                 300
   ctg gtg ctg gag cct gcc cgg aag gaa cac agt ggg cgc tat gaa tgt    960
   Leu Val Leu Glu Pro Ala Arg Lys Glu His Ser Gly Arg Tyr Glu Cys
   305                 310                 315                 320
   cag ggc ctg gac ttg gac acc atg ata tcg ctg ctg agt gaa cca cag   1008
   Gln Gly Leu Asp Leu Asp Thr Met Ile Ser Leu Leu Ser Glu Pro Gln
                   325                 330                 335
   gaa cta ctg gtg aac tat gtg tct gac gtc cga gtg agt ccc gca gcc   1056
   Glu Leu Leu Val Asn Tyr Val Ser Asp Val Arg Val Ser Pro Ala Ala
               340                 345                 350
   cct gag aga cag gaa ggc agc agc ctc acc ctg acc tgt gag gca gag 1104
   Pro Glu Arg Gln Glu Gly Ser Ser Leu Thr Leu Thr Cys Glu Ala Glu
   355 360 365
   agt agc cag gac ctc gag ttc cag tgg ctg aga gaa gag aca ggc cag 1152
   Ser Ser Gln Asp Leu Glu Phe Gln Trp Leu Arg Glu Glu Thr Gly Gln
   370 375 380
   gtg ctg gaa agg ggg cct gtg ctt cag ttg cat gac ctg aaa cgg gag 1200
   Val Leu Glu Arg Gly Pro Val Leu Gln Leu His Asp Leu Lys Arg Glu
   385 390 395 400
   gca gga ggc ggc tat cgc tgc gtg gcg tct gtg ccc agc ata ccc gga 1248
   Ala Gly Gly Gly Tyr Arg Cys Val Ala Ser Val Pro Ser Ile Pro Gly
   405 410 415
   ctg aac cgc aca cag ctg gtc aac gtg gcc att ttt ggc ccc cct tgg 1296
   Leu Asn Arg Thr Gln Leu Val Asn Val Ala Ile Phe Gly Pro Pro Trp
   420 425 430
   atg gca ttc aag gag agg aag gtg tgg gtg aaa gag aat atg gtg ttg 1344
   Met Ala Phe Lys Glu Arg Lys Val Trp Val Lys Glu Asn Met Val Leu
   435 440 445
   aat ctg tct tgt gaa gcg tca ggg cac ccc cgg ccc acc atc tcc tgg 1392
   Asn Leu Ser Cys Glu Ala Ser Gly His Pro Arg Pro Thr Ile Ser Trp
   450 455 460
   aac gtc aac ggc acg gca agt gaa caa gac caa gat cca cag cga gtc 1440
   Asn Val Asn Gly Thr Ala Ser Glu Gln Asp Gln Asp Pro Gln Arg Val
   465 470 475 480
   ctg agc acc ctg aat gtc ctc gtg acc ccg gag ctg ttg gag aca ggt 1488
   Leu Ser Thr Leu Asn Val Leu Val Thr Pro Glu Leu Leu Glu Thr Gly
   485 490 495
   gtt gaa tgc acg gcc tcc aac gac ctg ggc aaa aac acc agc atc ctc 1536
   Val Glu Cys Thr Ala Ser Asn Asp Leu Gly Lys Asn Thr Ser Ile Leu
   500 505 510
   ttc ctg gag ctg gtc aat tta acc acc ctc aca cca gac tcc aac aca 1584
   Phe Leu Glu Leu Val Asn Leu Thr Thr Leu Thr Pro Asp Ser Asn Thr
   515 520 525
   acc act ggc ctc agc act tcc act gcc agt cct cat acc aga gcc aac 1632
   Thr Thr Gly Leu Ser Thr Ser Thr Ala Ser Pro His Thr Arg Ala Asn
   530 535 540
   agc acc tcc aca gag aga aag ctg ccg gag ccg gag agc cgg ggc gtg 1680
   Ser Thr Ser Thr Glu Arg Lys Leu Pro Glu Pro Glu Ser Arg Gly Val
   545 550 555 560
   gtc atc gtg gct gtg att gtg tgc atc ctg gtc ctg gcg gtg ctg ggc 1728
   Val Ile Val Ala Val Ile Val Cys Ile Leu Val Leu Ala Val Leu Gly
   565 570 575
   gct gtc ctc tat ttc ctc tat aag aag ggc aag ctg ccg tgc agg cgc 1776
   Ala Val Leu Tyr Phe Leu Tyr Lys Lys Gly Lys Leu Pro Cys Arg Arg
   580 585 590
   tca ggg aag cag gag atc acg ctg ccc ccg tct cgt aag agc gaa ctt 1824
   Ser Gly Lys Gln Glu Ile Thr Leu Pro Pro Ser Arg Lys Ser Glu Leu
   595 600 605
   gta gtt gaa gtt aag tca gat aag ctc cca gaa gag atg ggc ctc ctg 1872
   Val Val Glu Val Lys Ser Asp Lys Leu Pro Glu Glu Met Gly Leu Leu
   610 615 620
   cag ggc agc agc ggt gac aag agg gct ccg gga gac cag gga gag aaa 1920
   Gln Gly Ser Ser Gly Asp Lys Arg Ala Pro Gly Asp Gln Gly Glu Lys
   625 630 635 640
   tac atc gat ctg agg cat tagccccgaa at 1950
   Tyr Ile Asp Leu Arg His
   645
<210> 2
   <211> 646
   <212> PRT
   <213> Homo sapiens
<400> 2
   Met Gly Leu Pro Arg Leu Val Cys Ala Phe Leu Leu Ala Ala Cys Cys
   1 5 10 15
   Cys Cys Leu Arg Val Ala Gly Val Pro Gly Glu Ala Glu Gln Pro Ala
   20 25 30
   Pro Glu Leu Val Glu Val Glu Val Gly Ser Thr Ala Leu Leu Lys Cys
   35 40 45
   Gly Leu Ser Gln Ser Gln Gly Asn Leu Ser His Val Asp Trp Phe Ser
   50 55 60
   Val His Lys Glu Lys Arg Thr Leu Ile Phe Arg Val Arg Gln Gly Gln
   65 70 75 80
   Gly Gln Ser Glu Pro Gly Glu Tyr Glu Gln Arg Leu Ser Leu Gln Asp
   85 90 95
   Arg Gly Ala Thr Leu Ala Leu Thr Gln Val Thr Pro Gln Asp Glu Arg
   100 105 110
   Ile Phe Leu Cys Gln Gly Lys Arg Pro Arg Ser Gln Glu Tyr Arg Ile
   115 120 125
   Gln Leu Arg Val Tyr Lys Ala Pro Glu Glu Pro Asn Ile Gln Val Asn
   130 135 140
   Pro Leu Gly Ile Pro Val Asn Ser Lys Glu Pro Glu Glu Val Ala Thr
   145 150 155 160
   Cys Val Gly Arg Asn Gly Tyr Pro Ile Pro Gln Val Ile Trp Tyr Lys
   165 170 175
   Asn Gly Arg Pro Leu Lys Glu Glu Lys Asn Arg Val His Ile Gln Ser
   180 185 190
   Ser Gln Thr Val Glu Ser Ser Gly Leu Tyr Thr Leu Gln Ser Ile Leu
   195 200 205
   Lys Ala Gln Leu Val Lys Glu Asp Lys Asp Ala Gln Phe Tyr Cys Glu
   210 215 220
   Leu Asn Tyr Arg Leu Pro Ser Gly Asn His Met Lys Glu Ser Arg Glu
   225 230 235 240
   Val Thr Val Pro Val Phe Tyr Pro Thr Glu Lys Val Trp Leu Glu Val
   245 250 255
   Glu Pro Val Gly Met Leu Lys Glu Gly Asp Arg Val Glu Ile Arg Cys
   260 265 270
   Leu Ala Asp Gly Asn Pro Pro Pro His Phe Ser Ile Ser Lys Gln Asn
   275 280 285
   Pro Ser Thr Arg Glu Ala Glu Glu Glu Thr Thr Asn Asp Asn Gly Val
   290 295 300
   Leu Val Leu Glu Pro Ala Arg Lys Glu His Ser Gly Arg Tyr Glu Cys
   305 310 315 320
   Gln Gly Leu Asp Leu Asp Thr Met Ile Ser Leu Leu Ser Glu Pro Gln
   325 330 335
   Glu Leu Leu Val Asn Tyr Val Ser Asp Val Arg Val Ser Pro Ala Ala
   340 345 350
   Pro Glu Arg Gln Glu Gly Ser Ser Leu Thr Leu Thr Cys Glu Ala Glu
   355 360 365
   Ser Ser Gln Asp Leu Glu Phe Gln Trp Leu Arg Glu Glu Thr Gly Gln
   370 375 380
   Val Leu Glu Arg Gly Pro Val Leu Gln Leu His Asp Leu Lys Arg Glu
   385 390 395 400
   Ala Gly Gly Gly Tyr Arg Cys Val Ala Ser Val Pro Ser Ile Pro Gly
   405 410 415
   Leu Asn Arg Arg Gln Leu Val Asn Val Ala Ile Phe Gly Pro Pro Trp
   420 425 430
   Met Ala Phe Lys Glu Arg Lys Val Trp Val Lys Glu Asn Met Val Leu
   435 440 445
   Asn Leu Ser Cys Glu Ala Ser Gly His Pro Arg Pro Thr Ile Ser Trp
   450 455 460
   Asn Val Asn Gly Thr Ala Ser Glu Gln Asp Gln Asp Pro Gln Arg Val
   465 470 475 480
   Leu Ser Thr Leu Asn Val Leu Val Thr Pro Glu Leu Leu Glu Thr Gly
   485 490 495
   Val Glu Cys Thr Ala Ser Asn Asp Leu Gly Lys Asn Thr Ser Ile Leu
   500 505 510
   Phe Leu Glu Leu Val Asn Leu Thr Thr Leu Thr Pro Asp Ser Asn Thr
   515 520 525
   Thr Thr Gly Leu Ser Thr Ser Thr Ala Ser Pro His Thr Arg Ala Asn
   530 535 540
   Ser Thr Ser Thr Glu Arg Lys Leu Pro Glu Pro Glu Ser Arg Gly Val
   545 550 555 560
   Val Ile Val Ala Val Ile Val Cys Ile Leu Val Leu Ala Val Leu Gly
   565 570 575
   Ala Val Leu Tyr Phe Leu Tyr Lys Lys Gly Lys Leu Pro Cys Arg Arg
   580 585 590
   Ser Gly Lys Gln Glu Ile Thr Leu Pro Pro Ser Ala Lys Ser Glu Leu
   595 600 605
   Val Val Glu Val Lys Ser Asp Lys Leu Pro Glu Glu Met Gly Leu Leu
   610 615 620
   Gln Gly Ser Ser Gly Asp Lys Arg Ala Pro Gly Asp Gln Gly Glu Lys
   625 630 635 640
   Tyr Ile Asp Leu Arg His
   645
<210> 3
   <211> 1960
   <212> DNA
   <213> Homo sapiens
<220>
   <221> CDS
   <222> (8)..(1945)
<400> 3
   gggaagc atg ggg ctt ccc agg ctg gtc tgc gcc ttc ttg ctc gcc gcc 49
   Met Gly Leu Pro Arg Leu Val Cys Ala Phe Leu Leu Ala Ala
   1 5 10
   tgc tgc tgc tgt cct cgc gtc gcg ggt gtg ccc gga gag gct gag cag 97
   Cys Cys Cys Cys Pro Arg Val Ala Gly Val Pro Gly Glu Ala Glu Gln
   15 20 25 30
   cct gcg cct gag ctg gtg gag gtg gaa gtg ggc agc aca gcc ctt ctg 145
   Pro Ala Pro Glu Leu Val Glu Val Glu Val Gly Ser Thr Ala Leu Leu
   35 40 45
   aag tgc ggc ctc tcc cag tcc caa ggc aac ctc agc cat gtc gac tgg 193
   Lys Cys Gly Leu Ser Gln Ser Gln Gly Asn Leu Ser His Val Asp Trp
   50 55 60
   ttt tct gtc cac aag gag aag cgg acg ctc atc ttc cgt gtg cgc cag 241
   Phe Ser Val His Lys Glu Lys Arg Thr Leu Ile Phe Arg Val Arg Gln
   65 70 75
   ggc cag ggc cag agc gaa cct ggg gag tac gag cag cgg ctc agc ctc 289
   Gly Gln Gly Gln Ser Glu Pro Gly Glu Tyr Glu Gln Arg Leu Ser Leu
   80 85 90
   cag gac aga ggg gct act ctg gcc ctg act caa gtc acc ccc caa gac   337
   Gln Asp Arg Gly Ala Thr Leu Ala Leu Thr Gln Val Thr Pro Gln Asp
    95                  100                 105                 110
   gag cgc atc ttc ttg tgc cag ggc aag cgc cct cgg tcc cag gag tac   385
   Glu Arg Ile Phe Leu Cys Gln Gly Lys Arg Pro Arg Ser Gln Glu Tyr
                   115                 120                 125
   cgc atc cag ctc cgc gtc tac aaa gct ccg gag gag cca aac atc cag   433
   Arg Ile Gln Leu Arg Val Tyr Lys Ala Pro Glu Glu Pro Asn Ile Gln
               130                 135                 140
   gtc aac ccc ctg ggc atc cct gtg aac agt aag gag cct gag gag gtc   481
   Val Asn Pro Leu Gly Ile Pro Val Asn Ser Lys Glu Pro Glu Glu Val
           145                 150                 155
   gct acc tgt gta ggg agg aac ggg tac ccc att cct caa gtc atc tgg   529
   Ala Thr Cys Val Gly Arg Asn Gly Tyr Pro Ile Pro Gln Val Ile Trp
       160                 165                 170
   tac aag aat ggc cgg cct ctg aag gag gag aag aac cgg gtc cac att   577
   Tyr Lys Asn Gly Arg Pro Leu Lys Glu Glu Lys Asn Arg Val His Ile
   175                 180                 185                 190
   cag tcg tcc cag act gtg gag tcg agt ggt ttg tac acc ttg cag agt   625
   Gln Ser Ser Gln Thr Val Glu Ser Ser Gly Leu Tyr Thr Leu Gln Ser
                   195                 200                 205
   att ctg aag gca cag ctg gtt aaa gaa gac aaa gat gcc cag ttt tac   673
   Ile Leu Lys Ala Gln Leu Val Lys Glu Asp Lys Asp Ala Gln Phe Tyr
               210                 215                 220
   tgt gag ctc aac tac cgg ctg ccc agt ggg aac cac atg aag gag tcc   721
   Cys Glu Leu Asn Tyr Arg Leu Pro Ser Gly Asn His Met Lys Glu Ser
           225                 230                 235
   agg gaa gtc acc gtc cct gtt ttc tac ccg aca gaa aaa gtg tgg ctg   769
   Arg Glu Val Thr Val Pro Val Phe Tyr Pro Thr Glu Lys Val Trp Leu
       240                 245                 250
   gaa gtg gag ccc gtg gga atg ctg aag gaa ggg gac cgc gtg gaa atc 817
   Glu Val Glu Pro Val Gly Met Leu Lys Glu Gly Asp Arg Val Glu Ile
   255 260 265 270
   agg tgt ttg gct gat ggc aac cct cca cca cac ttc agc atc agc aag 865
   Arg Cys Leu Ala Asp Gly Asn Pro Pro Pro His Phe Ser Ile Ser Lys
   275 280 285
   cag aac ccc agc acc agg gag gca gag gaa gag aca acc aac gac aac 913
   Gln Asn Pro Ser Thr Arg Glu Ala Glu Glu Glu Thr Thr Asn Asp Asn
   290 295 300
   ggg gtc ctg gtg ctg gag cct gcc cgg aag gaa cac agt ggg cgc tat 961
   Gly Val Leu Val Leu Glu Pro Ala Arg Lys Glu His Ser Gly Arg Tyr
   305 310 315
   gaa tgt cag gcc tgg aac ttg gac acc atg ata tcg ctg ctg agt gaa 1009
   Glu Cys Gln Ala Trp Asn Leu Asp Thr Met Ile Ser Leu Leu Ser Glu
   320 325 330
   cca cag gaa cta ctg gtg aac tat gtg tct gac gtc cga gtg agt ccc   1057
   Pro Gln Glu Leu Leu Val Asn Tyr Val Ser Asp Val Arg Val Ser Pro
   335                 340                 345                 350
   gca gcc cct gag aga cag gaa ggc agc agc ctc acc ctg acc tgt gag   1105
   Ala Ala Pro Glu Arg Gln Glu Gly Ser Ser Leu Thr Leu Thr Cys Glu
                   355                 360                 365
   gca gag agt agc cag gac ctc gag ttc cag tgg ctg aga gaa gag aca   1153
   Ala Glu Ser Ser Gln Asp Leu Glu Phe Gln Trp Leu Arg Glu Glu Thr
               370                 375                 380
   gac cag gtg ctg gaa agg ggg cct gtg ctt cag ttg cat gac ctg aaa   1201
   Asp Gln Val Leu Glu Arg Gly Pro Val Leu Gln Leu His Asp Leu Lys
           385                 390                 395
   cgg gag gca gga ggc ggc tat cgc tgc gtg gcg tct gtg ccc agc ata   1249
   Arg Glu Ala Gly Gly Gly Tyr Arg Cys Val Ala Ser Val Pro Ser Ile
       400                 405                 410
   ccc ggc ctg aac cgc aca cag ctg gtc aag ctg gcc att ttt ggc ccc 1297
   Pro Gly Leu Asn Arg Thr Gln Leu Val Lys Leu Ala Ile Phe Gly Pro
   415 420 425 430
   cct tgg atg gca ttc aag gag agg aag gtg tgg gtg aaa gag aat atg 1345
   Pro Trp Met Ala Phe Lys Glu Arg Lys Val Trp Val Lys Glu Asn Met
   435 440 445
   gtg ttg aat ctg tct tgt gaa gcg tca ggg cac ccc cgg ccc acc atc 1393
   Val Leu Asn Leu Ser Cys Glu Ala Ser Gly His Pro Arg Pro Thr Ile
   450 455 460
   tcc tgg aac gtc aac ggc acg gca agt gaa caa gac caa gat cca cag 1441
   Ser Trp Asn Val Asn Gly Thr Ala Ser Glu Gln Asp Gln Asp Pro Gln
   465 470 475
   cga gtc ctg agc acc ctg aat gtc ctc gtg acc ccg gag ctg ttg gag 1489
   Arg Val Leu Ser Thr Leu Asn Val Leu Val Thr Pro Glu Leu Leu Glu
   480 485 490
   aca ggt gtt gaa tgc acg gcc tcc aac gac ctg ggc aaa aac acc agc   1537
   Thr Gly Val Glu Cys Thr Ala Ser Asn Asp Leu Gly Lys Asn Thr Ser
   495                 500                 505                 510
   atc ctc ttc ctg gag ctg gtc aat tta acc acc ctc aca cca gac tcc   1585
   Ile Leu Phe Leu Glu Leu Val Asn Leu Thr Thr Leu Thr Pro Asp Ser
                   515                 520                 525
   aac aca acc act ggc ctc agc act tcc act gcc agt cct cat acc aga   1633
   Asn Thr Thr Thr Gly Leu Ser Thr Ser Thr Ala Ser Pro His Thr Arg
               530                 535                 540
   gcc aac agc acc tcc aca gag aga aag ctg ccg gag ccg gag agc cgg   1681
   Ala Asn Ser Thr Ser Thr Glu Arg Lys Leu Pro Glu Pro Glu Ser Arg
           545                 550                 555
   ggc gtg gtc atc gtg gct gtg att gtg tgc atc ctg gtc ctg gcg gtg   1729
   Gly Val Val Ile Val Ala Val Ile Val Cys Ile Leu Val Leu Ala Val
       560                 565                 570
   ctg ggc gct gtc ctc tat ttc ctc tat aag aag ggc aag ctg ccg tgc   1777
   Leu Gly Ala Val Leu Tyr Phe Leu Tyr Lys Lys Gly Lys Leu Pro Cys
   575                 580                 585                 590
   agg cgc tca ggg aag cag gag atc acg ctg ccc ccg tct cgt aag acc   1825
   Arg Arg Ser Gly Lys Gln Glu Ile Thr Leu Pro Pro Ser Arg Lys Thr
                   595                 600                 605
   gaa ctt gta gtt gaa gtt aag tca gat aag ctc cca gaa gag atg ggc   1873
   Glu Leu Val Val Glu Val Lys Ser Asp Lys Leu Pro Glu Glu Met Gly
               610                 615                 620
   ctc ctg cag ggc agc agc ggt gac aag agg gct ccg gga gac cag gga   1921
   Leu Leu Gln Gly Ser Ser Gly Asp Lys Arg Ala Pro Gly Asp Gln Gly
           625                 630                 635
   gag aaa tac atc gat ctg agg cat tagccccgaa tcact                  1960
   Glu Lys Tyr Ile Asp Leu Arg His
       640                 645
<210> 4
   <211> 646
   <212> PRT
   <213> Homo sapiens
<400> 4
   Met Gly Leu Pro Arg Leu Val Cys Ala Phe Leu Leu Ala Ala Cys Cys
   1 5 10 15
   Cys Cys Pro Arg Val Ala Gly Val Pro Gly Glu Ala Glu Gln Pro Ala
   20 25 30
   Pro Glu Leu Val Glu Val Glu Val Gly Ser Thr Ala Leu Leu Lys Cys
   35 40 45
   Gly Leu Ser Gln Ser Gln Gly Asn Leu Ser His Val Asp Trp Phe Ser
   50 55 60
   Val His Lys Glu Lys Arg Thr Leu Ile Phe Arg Val Arg Gln Gly Gln
   65 70 75 80
   Gly Gln Ser Glu Pro Gly Glu Tyr Glu Gln Arg Leu Ser Leu Gln Asp
   85 90 95
   Arg Gly Ala Thr Leu Ala Leu Thr Gln Val Thr Pro Gln Asp Glu Arg
   100 105 110
   Ile Phe Leu Cys Gln Gly Lys Arg Pro Arg Ser Gln Glu Tyr Arg Ile
   115 120 125
   Gln Leu Arg Val Tyr Lys Ala Pro Glu Glu Pro Asn Ile Gln Val Asn
   130 135 140
   Pro Leu Gly Ile Pro Val Asn Ser Lys Glu Pro Glu Glu Val Ala Thr
   145 150 155 160
   Cys Val Gly Arg Asn Gly Tyr Pro Ile Pro Gln Val Ile Trp Tyr Lys
   165 170 175
   Asn Gly Arg Pro Leu Lys Glu Glu Lys Asn Arg Val His Ile Gln Ser
   180 185 190
   Ser Gln Thr Val Glu Ser Ser Gly Leu Tyr Thr Leu Gln Ser Ile Leu
   195 200 205
   Lys Ala Gln Leu Val Lys Glu Asp Lys Asp Ala Gln Phe Tyr Cys Glu
   210 215 220
   Leu Asn Tyr Arg Leu Pro Ser Gly Asn His Met Lys Glu Ser Arg Glu
   225 230 235 240
   Val Thr Val Pro Val Phe Tyr Pro Thr Glu Lys Val Trp Leu Glu Val
   245 250 255
   Glu Pro Val Gly Met Leu Lys Glu Gly Asp Arg Val Glu Ile Arg Cys
   260 265 270
   Leu Ala Asp Gly Asn Pro Pro Pro His Phe Ser Ile Ser Lys Gln Asn
   275 280 285
   Pro Ser Thr Arg Glu Ala Glu Glu Glu Thr Thr Asn Asp Asn Gly Val
   290 295 300
   Leu Val Leu Glu Pro Ala Arg Lys Glu His Ser Gly Arg Tyr Glu Cys
   305 310 315 320
   Gln Ala Trp Asn Leu Asp Thr Met Ile Ser Leu Leu Ser Glu Pro Gln
   325 330 335
   Glu Leu Leu Val Asn Tyr Val Ser Asp Val Arg Val Ser Pro Ala Ala
   340 345 350
   Pro Glu Arg Gln Glu Gly Ser Ser Leu Thr Leu Thr Cys Glu Ala Glu
   355 360 365
   Ser Ser Gln Asp Leu Glu Phe Gln Trp Leu Arg Glu Glu Thr Asp Gln
   370 375 380
   Val Leu Glu Arg Gly Pro Val Leu Gln Leu His Asp Leu Lys Arg Glu
   385 390 395 400
   Ala Gly Gly Gly Tyr Arg Cys Val Ala Ser Val Pro Ser Ile Pro Gly
   405 410 415
   Leu Asn Arg Thr Gln Leu Val Lys Leu Ala Ile Phe Gly Pro Pro Trp
   420 425 430
   Met Ala Phe Lys Glu Arg Lys Val Trp Val Lys Glu Asn Met Val Leu
   435 440 445
   Asn Leu Ser Cys Glu Ala Ser Gly His Pro Arg Pro Thr Ile Ser Trp
   450 455 460
   Asn Val Asn Gly Thr Ala Ser Glu Gln Asp Gln Asp Pro Gln Arg Val
   465 470 475 480
   Leu Ser Thr Leu Asn Val Leu Val Thr Pro Glu Leu Leu Glu Thr Gly
   485 490 495
   Val Glu Cys Thr Ala Ser Asn Asp Leu Gly Lys Asn Thr Ser Ile Leu
   500 505 510
   Phe Leu Glu Leu Val Asn Leu Thr Thr Leu Thr Pro Asp Ser Asn Thr
   515 520 525
   Thr Thr Gly Leu Ser Thr Ser Thr Ala Ser Pro His Thr Arg Ala Asn
   530 535 540
   Ser Thr Ser Thr Glu Arg Lys Leu Pro Glu Pro Glu Ser Arg Gly Val
   545 550 555 560
   Val Ile Val Ala Val Ile Val Cys Ile Leu Val Leu Ala Val Leu Gly
   565 570 575
   Ala Val Leu Tyr Phe Leu Tyr Lys Lys Gly Lys Leu Pro Cys Arg Arg
   580 585 590
   Ser Gly Lys Gln Glu Ile Thr Leu Pro Pro Ser Arg Lys Thr Glu Leu
   595 600 605
   Val Val Glu Val Lys Ser Asp Lys Leu Pro Glu Glu Met Gly Leu Leu
   610 615 620
   Gln Gly Ser Ser Gly Asp Lys Arg Ala Pro Gly Asp Gln Gly Glu Lys
   625 630 635 640
   Tyr Ile Asp Leu Arg His
   645
<210> 5
   <211> 1962
   <212> DNA
   <213> Artificial Sequence
<220>
   <223> Description of Artificial Sequence:HUMAN MUC18 cDNA, AS MODIFIED TO FACILITATE CLONING
<400> 5
   ctcggatcca tggggcttcc caggctggtc tgcgccttct tgctcgccgc ctgctgctgc 60
   tgtcctcgcg tcgcgggtgt gcccggagag gctgagcagc ctgcgcctga gctggtggag 120
   gtggaagtgg gcagcacagc ccttctgaag tgcggcctct cccagtccca aggcaacctc 180
   agccatgtcg actggttttc tgtccacaag gagaagcgga cgctcatctt ccgtgtgcgc 240
   cagggccagg gccagagcga acctggggag tacgagcagc ggctcagcct ccaggacaga 300
   ggggctactc tggccctgac tcaagtcacc ccccaagacg agcgcatctt cttgtgccag 360
   ggcaagcgcc ctcggtccca ggagtaccgc atccagctcc gcgtctacaa agctccggag 420
   gagccaaaca tccaggtcaa ccccctgggc atccctgtga acagtaagga gcctgaggag 480
   gtcgctacct gtgtagggag gaacgggtac cccattcctc aagtcatctg gtacaagaat 540
   ggccggcctc tgaaggagga gaagaaccgg gtccacattc agtcgtccca gactgtggag 600
   tcgagtggtt tgtacacctt gcagagtatt ctgaaggcac agctggttaa agaagacaaa 660
   gatgcccagt tttactgtga gctcaactac cggctgccca gtgggaacca catgaaggag 720
   tccagggaag tcaccgtccc tgttttctac ccgacagaaa aagtgtggct ggaagtggag 780
   cccgtgggaa tgctgaagga aggggaccgc gtggaaatca ggtgtttggc tgatggcaac 840
   cctccaccac acttcagcat cagcaagcag aaccccagca ccagggaggc agaggaagag 900
   acaaccaacg acaacggggt cctggtgctg gagcctgccc ggaaggaaca cagtgggcgc 960
   tatgaatgtc aggcctggaa cttggacacc atgatatcgc tgctgagtga accacaggaa 1020
   ctactggtga actatgtgtc tgacgtccga gtgagtcccg cagcccctga gagacaggaa 1080
   ggcagcagcc tcaccctgac ctgtgaggca gagagtagcc aggacctcga gttccagtgg 1140
   ctgagagaag agacagacca ggtgctggaa agggggcctg tgcttcagtt gcatgacctg 1200
   aaacgggagg caggaggcgg ctatcgctgc gtggcgtctg tgcccagcat acccggcctg 1260
   aaccgcacac agctggtcaa gctggccatt tttggccccc cttggatggc attcaaggag 1320
   aggaaggtgt gggtgaaaga gaatatggtg ttgaatctgt cttgtgaagc gtcagggcac 1380
   ccccggccca ccatctcctg gaacgtcaac ggcacggcaa gtgaacaaga ccaagatcca 1440
   cagcgagtcc tgagcaccct gaatgtcctc gtgaccccgg agctgttgga gacaggtgtt 1500
   gaatgcacgg cctccaacga cctgggcaaa aacaccagca tcctcttcct ggagctggtc 1560
   aatttaacca ccctcacacc agactccaac acaaccactg gcctcagcac ttccactgcc 1620
   agtcctcata ccagagccaa cagcacctcc acagagagaa agctgccgga gccggagagc 1680
   cggggcgtgg tcatcgtggc tgtgattgtg tgcatcctgg tcctggcggt gctgggcgct 1740
   gtcctctatt tcctctataa gaagggcaag ctgccgtgca ggcgctcagg gaagcaggag 1800
   atcacgctgc ccccgtctcg taagaccgaa cttgtagttg aagttaagtc agataagctc 1860
   ccagaagaga tgggcctcct gcagggcagc agcggtgaca agagggctcc gggagaccag 1920
   ggagagaaat acatcgatct gaaggcatta gccccgaatc at 1962
<210> 6
   <211> 28
   <212> DNA
   <213> Artificial Sequence
<220>
   <223> Description of Artificial
   Sequence:OLIGONUCLEOTIDE, FOR SUBCLONING HUMAN
   MUC18 FRAGMENT
<400> 6
   ggatcccagc tggttaaaga agacaaag 28
<210> 7
   <211> 27
   <212> DNA
   <213> Artificial Sequence
<220>
   <223> Description of Artificial
   Sequence:OLIGONUCLEOTIDE, FOR SUBCLONING HUMAN
   MUC18
<400> 7
   ctggaactcg aggtcctggc tactctc 27
<210> 8
   <211> 5
   <212> PRT
   <213> Artificial Sequence
<220>
   <223> Description of Artificial Sequence:AMINO ACID
   SEQUENCE ENCODED BY PORTION OF PGEX-6P-1 VECTOR
<400> 8
   Gly Pro Leu Gly Ser
   1               5
<210> 9
   <211> 27
   <212> DNA
   <213> Artificial Sequence
<220>
   <223> Description of Artificial
   Sequence:OLIGONUCLEOTIDE, SEQUENCE CORRESPONDS TO
   HUMAN MUC18
<400> 9
   ctcgggatcc atggggcttc ccaggct                                          27
<210> 10
   <211> 25
   <212> DNA
   <213> Artificial Sequence
<220>
   <223> Description of Artificial
   Sequence:OLIGONUCLEOTIDE, SEQUENCE CORRESPONDS TO
   HUMAN MUC18
<400> 10
   tcggggctaa tgcctcagat cgatg                                            25
<210> 11
   <211> 81
   <212> DNA
   <213> Artificial Sequence
<220>
   <223> Description of Artificial Sequence:JUNCTION
   SEQUENCE FOR MCU18 CLONED INSERT
<400> 11
   ctggaagttc tgttccaggg gcccctggga tccccggaat tcccgggtcg actcgagcgg 60
   ccgcatcgtg actgactgac g 81
<210> 12
   <211> 24
   <212> PRT
   <213> Artificial Sequence
<220>
   <223> Description of Artificial Sequence:JUNCTION
   SEQUENCE FOR CLONED MUC18 INSERT
<400> 12
   Leu Glu Val Leu Phe Gln Gly Pro Leu Gly Ser Pro Glu Phe Pro Gly
   1 5 10 15
   Arg Leu Glu Arg Pro His Arg Asp
   20
<210> 13
   <211> 81
   <212> DNA
   <213> Artificial Sequence
<220>
   <223> Description of Artificial Sequence:JUNCTION
   SEQUENCE FOR CLONED MUC18 INSERT
<400> 13
   ctggaagttc tgttccaggg gcccctggga tccccaggaa ttcccgggtc gactcgagcg 60
   gccgcatcgt gactgactga c 81
<210> 14
   <211> 23
   <212> PRT
   <213> Artificial Sequence
<220>
   <223> Description of Artificial Sequence:JUNCTION
   SEQUENCE IN FUSION PROTEIN
<400> 14
   Leu Glu Val Leu Phe Gln Gly Pro Leu Gly Ser Pro Gly Ile Pro Gly
   1 5 10 15
   Ser Thr Arg Ala Ala Ala Ser
   20
<210> 15
   <211> 78
   <212> DNA
   <213> Artificial Sequence
<220>
   <223> Description of Artificial Sequence:JUNCTION
   SEQUENCE IN VECTOR WITH MCU18 CLONED INSERT
<400> 15
   ctggaagttc tgttccaggg gcccctggga tccccgaatt cccgggtcga ctcgagcggc 60
   cgcatcgtga ctgactga 78
<210> 16
   <211> 26
   <212> PRT
   <213> Artificial Sequence
<220>
   <223> Description of Artificial Sequence:JUNCTION OF
   FUSION PEPTIDE
<400> 16